# EUROPEAN PATENT APPLICATION

(11) **EP 1 428 877 A1**
(43) Date of publication of application: **16.06.2004**
(21) Application number: 02798663.7
(22) Date of filing: 13.09.2002
(51) Int. Cl.: C12N 15/09, C07K 14/47, C07K 16/18, C12N 5/10, C12Q 1/02, C12Q 1/68, C12P 21/08, A61K 38/16, A61P 35/00, A61P 43/00, A61P 1/16, A61P 9/00, A61P 3/00, A61P 13/08, A61P 13/02, G01N 33/53, G01N 33/50

(54) **NOVEL PROTEIN AND DNA THEREOF**

(30) Priority: 17.09.2001 JP 2001281992; 02.10.2001 JP 2001306873; 16.04.2002 JP 2002113279
(71) Applicant: Takeda Chemical Industries, Ltd., Osaka-shi, Osaka 541-0045 (JP)
(72) Inventor: NAKANISHI, Atsushi, Tsukuba-shi, Ibaraki 305-0025 (JP); UNO, Yumiko Takeda Matsushiro Residence 601, Tsukuba-shi, Ibaraki 305-0035 (JP); SAGIYA, Yoji Takeda Kasuga Haitsu 602, Tsukuba-shi, Ibaraki 305-0821 (JP)
(74) Representative: Rickard, Timothy Mark Adrian
(86) International application number: PCT/JP2002/009444
(87) International publication number: WO 2003/025168

(57) **Abstract**

A novel protein having a dicarboxylic acid transport activity, a DNA encoding this protein, a method of screening a compound that promotes or inhibits the activity of the protein, compounds obtained by the screening method, etc. are provided.

The protein of the present invention is useful as, for example, a diagnostic marker for liver diseases, prostatic diseases, spleen diseases, kidney diseases, metabolic diseases, circulatory diseases, cancer, etc. Compounds that promote or inhibit the activity of the protein can be used, e.g., for the prevention/treatment of these diseases.

## Description

### FIELD OF THE INVENTION

The present invention provides a novel sodium-dependent dicarboxylate transporter (NaDC) protein, a DNA encoding the protein, a method of screening a compound that promotes or inhibits the activity of the protein, compounds obtained by the screening method, etc.

### BACKGROND ART

Di- or tricarboxylic acids including succinic acid and citric acid play a primary role as intermediates in the TCA cycle. What is engaged in intracellular and extracellular transport of these acids is sodium-dependent dicarboxylate transporter (NaDC). To date 2 types of NaDC are reported in human. In these transporters, NaDCl has a low affinity to substrate and is expressed in the kidney or the small intestine, but not found in the liver. On the other hand, NaDC3 has a high affinity to substrate so that it is expressed in the proximal tubule, liver cells, brain, etc. These NaDCs are considered to play an important role in transporting citric acid in the kidney, suggesting that they might be responsible for urinary calculus formation.
Patent Reference 1: US 2002/0019028
Non-Patent Reference 1: American Journal of Physiology, 270, F642 (1996)
Non-Patent Reference 2: American Journal of Physiology, 279, F482 (2000)

### DISCLOSURE OF THE INVENTION

NaDC is considered to play a critical role in the metabolism of a dicarboxylic acid or a tricarboxylic acid but its detailed mechanism or possible association with diseases remains unclear. To throw light on the detailed substrate specificity of NaDC or its role in the metabolic response will lead to development of curatives for diseases associated with dicarboxylic acid or tricarboxylic acid metabolism.

Also, NaDCl is a human gene having the highest homology to Indy, which takes part in aging of fly (Science, 290, 2137-2140, 2000). Since Indy transports a dicarboxylic acid or tricarboxylic acid (Biochem. J. Intermediate Publication. Published on 19 Aug 2002 as manuscript BJ20021132), it will lead to development of pharmaceuticals for preventing the progress of aging if it is clarified how the function of NaDC family is associated with aging.

In order to solve the problems described above, the present inventors made extensive studied and found a novel sodium-dependent dicarboxylate transporter (NaDC) protein. On an amino acid level, the protein shows a homology of 56% to human NaDCl, indicating that the protein can function as a sodium-dependent dicarboxylate transporter. In order to suppress the function or activity of the protein, it is considered to inhibit the transport of, e.g., a di- or tricarboxylic acid or prevent transcription of the protein thereby to reduce its expression level; in order to inactivate the function or activity of the protein, it is considered to promote the transport of, e.g., a di- or tricarboxylic acid, activate a promoter of the protein or stabilize mRNA, thereby to increase its expression level.

Based on these findings, the inventors have further made extensive investigations and come to accomplish the present invention.

### DISCLOSURE OF THE INVENTION

That is, the present invention provides the following features.
(1) A protein comprising the same or substantially the same amino acid sequence as the amino acid sequence represented by SEQ ID NO: 1 or SEQ ID NO: 23, or a salt thereof.
(2) A protein consisting of the amino acid sequence represented by SEQ ID NO: 1, or a salt thereof.
(3) A protein consisting of the amino acid sequence represented by SEQ ID NO: 23, or a salt thereof.
(4) A partial peptide of the protein according to (1), or a salt thereof.
(5) A polynucleotide containing a polynucleotide encoding the protein according to (1) or the partial peptide according to (4).
(6) A polynucleotide according to (5), which is DNA.
(7) A polynucleotide consisting of the base sequence represented by SEQ ID NO: 2 or SEQ ID NO: 22.
(8) A polynucleotide consisting of the base sequence represented by SEQ ID NO: 24 or SEQ ID NO: 36.
(9) A recombinant vector containing the polynucleotide according to (5).
(10) A transformant transformed with the recombinant vector according to (9).
(11) A method of manufacturing the protein according to (1) or the partial peptide according to (4), or a salt thereof, which comprises culturing the transformant according to (10), producing, accumulating and collecting the protein according to (1) or the partial peptide according to (4).
(12) A drug comprising the protein according to (1) or the partial peptide according to (4), or a salt of said protein or partial peptide.
(13) A drug comprising the polynucleotide according to (5).
(14) A diagnostic agent comprising the polynucleotide according to (5).
(15) An antibody to the protein according to (1) or the partial peptide according to (4), or a salt of said protein or partial peptide.
(16) A drug comprising the antibody according to (15).
(17) A diagnostic agent comprising the antibody according to (15).
(18) A polynucleotide comprising a base sequence complementary or substantially complementary to the polynucleotide according to (5) or a part of the base sequence.
(19) A drug comprising the polynucleotide according to (18).
(20) A method of screening a compound or its salt that promotes or inhibits the activity of the protein according to (1) or the partial peptide according to (4), or a salt thereof, which comprises using the protein according to (1) or the partial peptide according to (4), or a salt of said protein or partial peptide.
(21) A kit for screening a compound or its salt that promotes or inhibits the activity of the protein according to (1) or the partial peptide according to (4), or a salt thereof, comprising the protein according to (1) or the partial peptide according to (4), or a salt of said protein or partial peptide.
(22) A compound or its salt that promotes or inhibits the activity of the protein according to (1) or the partial peptide according to (4), or a salt thereof, which is obtainable using the screening method according to (20) or the screening kit according to (21).
(23) A drug comprising the compound or its salt according to (22).
(24) A method of screening a compound or its salt that promotes or inhibits expression of a gene for the protein according to (1), which comprises using the polynucleotide according to (5).
(25) A kit for screening a compound or its salt that promotes or inhibits expression of a gene for the protein according to (1), comprising the polynucleotide according to (5).
(26) A compound or its salt that promotes or inhibits expression of a gene for the protein according to (1), which is obtainable using the screening method according to (24) or the screening kit according to (25).
(27) A drug comprising the compound or its salt according to (26).
(28) A method of quantifying the protein according to (1), which comprises using the antibody according to (15).
(29) A diagnostic method for a disease associated with functions of the protein according to (1), which comprises using the quantification method according to (28).
(30) A method of screening a compound or its salt that promotes or inhibits expression of the protein according to (1), which comprises using the antibody according to (15).
(31) A kit for screening a compound or its salt that promotes or inhibits expression of the protein according to (1), comprising the antibody according to (15).
(32) A compound or its salt that promotes or inhibits expression of the protein according to (1), which is obtainable using the screening method according to (30) or the screening kit according to (31).
(33) A drug comprising the compound or its salt according to (32).
(34) A drug according to (13), (16), (19), (23), (27) or (33), which is an agent for the prevention/treatment of hepatic dysfunctions, prostate diseases, splenic diseases, renal diseases, metabolic diseases, circulatory disorders or cancer, or an agent for the prevention/retardation of aging.
(35) A diagnostic agent according to (14) or (17), which is a diagnostic agent for hepatic dysfunctions, prostate diseases, splenic diseases, renal diseases, metabolic diseases, circulatory disorders or cancer.
(36) A method for the prevention/treatment of hepatic dysfunctions, prostate diseases, splenic diseases, renal diseases, metabolic diseases, circulatory disorders or cancer, or for the prevention/retardation of aging, which comprises administering an effective dose of a compound or its salt according to (22), (26) or (32) to a mammal.
(37) Use of a compound or its salt according to(22), (26) or (32) for manufacturing an agent for the prevention/treatment of hepatic dysfunctions, prostate diseases, splenic diseases, renal diseases, metabolic diseases, circulatory disorders or cancer, or for the prevention/retardation of aging.

### BRIEF DESCRIPTION OF THE DRAWINGS

FIG. 1 shows a comparison in amino acid sequence between sodium-dependent dicarboxylate transporter NaDCl and human TCH169 (continued on FIG. 2).
FIG. 2 shows a comparison in amino acid sequence between sodium-dependent dicarboxylate transporter NaDCl and human TCH169 (continued from FIG. 1).
FIG. 3 shows expression levels of human TCH169 gene products in various tissues.
FIG. 4 shows a comparison in amino acid sequence between mouse TCH169 and human TCH169.
FIG. 5 shows expression levels of mouse TCH169 gene products in various tissues.

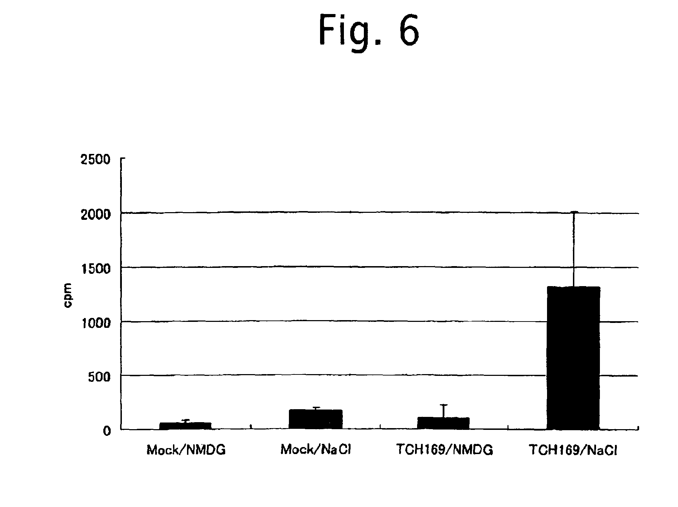

FIG. 6 shows the results obtained by measuring uptake of [1,5-¹⁴C] citric acid in human TCH169 expressing CHO cell line. The uptake is expressed in terms of count (cpm) per minute when the uptake of [1,5-¹⁴C] citric acid is performed for an hour, with a mean value of counts from independent 4 wells and its standard deviation. In the figure, vector pcDNA3.1(+)-inserted cells and human TCH169 expressing CHO cells are represented by Mock and TCH169, respectively; thus, the cells where the uptake of [1,5-¹⁴C] citric acid was performed using NaCl buffer are represented by Mock/NaCl and TCH169/NaCl, respectively and the cells where the uptake of [1,5-¹⁴C] citric acid was performed using NMDG buffer are represented by Mock/NMDG and TCH169/NMDG, respectively.

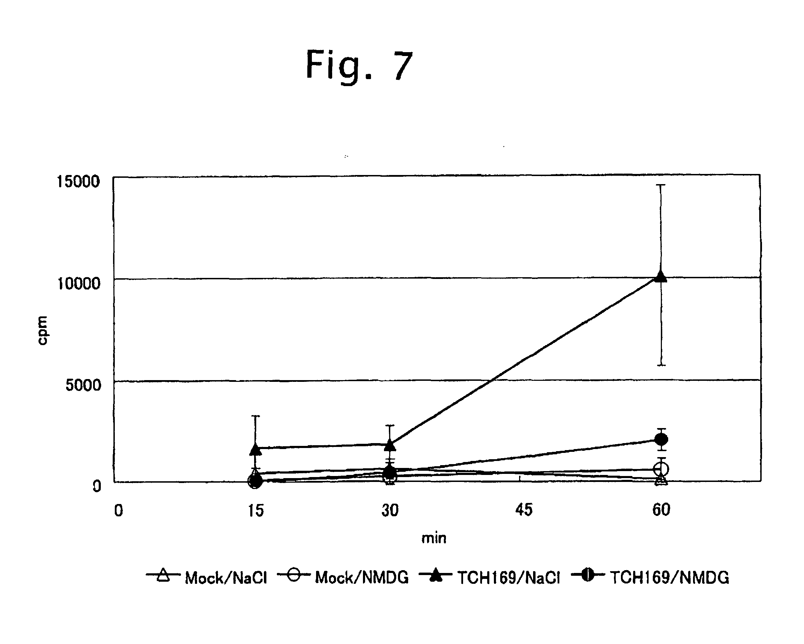

FIG. 7 shows the results obtained by measuring uptake of [1,5-¹⁴C] citric acid in human TCH169 expressing CHO cell line with passage of time. The uptake is expressed in terms of count (cpm) per minute when the uptake of [1,5-¹⁴C] citric acid is performed for 15 minutes, 30 minutes and an hour, with a mean value of counts from independent 4 wells and its standard deviation. In the figure, vector pcDNA3.1(+)-inserted cells and human TCH169 expressing CHO cells are represented by Mock and TCH169, respectively; thus, the cells where the uptake of [1,5-¹⁴C] citric acid was performed using NaCl buffer are represented by Mock/NaCl and TCH169/NaCl, respectively and the cells where the uptake of [1,5-¹⁴C] citric acid was performed using NMDG buffer are represented by Mock/NMDG and TCH169/NMDG, respectively.

### BEST MODE FOR CARRYING OUT THE INVENTION

The protein comprising the same or substantially the same amino acid sequence as the amino acid sequence represented by SEQ ID NO: 1 or SEQ ID NO: 23 (hereinafter referred to as the protein of the present invention or sometimes also referred to as the protein used in the present invention) may be any protein derived from any cells (e.g., hepatocytes, splenocytes, nerve cells, glial cells, β cells of pancreas, bone marrow cells, mesangial cells, Langerhans' cells, epidermic cells, epithelial cells, goblet cells, endothelial cells, smooth muscle cells, fibroblasts, fibrocytes, myocytes, fat cells, immune cells (e.g., macrophage, T cells, β cells, natural killer cells, mast cells, neutrophils, basophils, eosinophils, monocytes, etc.), megakaryocyte, synovial cells, chondrocytes, bone cells, osteoblasts, osteoclasts, mammary gland cells, hepatocytes or interstitial cells, the corresponding precursor cells, stem cells, cancer cells, etc.), hemocyte type cells, or any tissues where such cells are present, e.g., brain or any region of the brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gall-bladder, bone marrow, adrenal gland, skin, muscle, lung, gastrointestinal tract (e.g., large intestine and small intestine), blood vessel, heart, thymus, spleen, submandibular gland, peripheral blood, prostate, testis, ovary, placenta, uterus, bone, joint, skeletal muscle, etc., from human and other warm-blooded animals (e.g., guinea pigs, rats, mice, chicken, rabbits, swine, sheep, bovine, monkeys, etc.). The protein may also be a synthetic protein.

The amino acid sequence which has substantially the same amino acid sequence as that represented by SEQ ID NO: 1 includes an amino acid sequence having at least about 60% homology, preferably at least about 70% homology, more preferably at least about 80% homology, among others preferably at least about 90% homology and most preferably at least about 95% homology, to the amino acid sequence represented by SEQ ID NO: 1.

Examples of the protein which comprising substantially the same amino acid sequence as that represented by SEQ ID NO: 1 include a protein having substantially the same amino acid sequence as that represented by SEQ ID NO: 1 and having the activity substantially equivalent to the amino acid sequence represented by SEQ ID NO: 1, etc.

The amino acid sequence which has substantially the same amino acid sequence as that represented by SEQ ID NO: 23 includes an amino acid sequence having at least about 50% homology, preferably at least about 60% homology, more preferably at least about 70% homology, much more preferably at least about 80% homology, among others preferably at least about 90% homology and most preferably at least about 95% homology, to the amino acid sequence represented by SEQ ID NO: 23.

Examples of the protein which comprises substantially the same amino acid sequence as that represented by SEQ ID NO: 23 include a protein having substantially the same amino acid sequence as that represented by SEQ ID NO: 23 and having the activity substantially equivalent to the amino acid sequence represented by SEQ ID NO: 23, etc.

Examples of the substantially equivalent activity include the transport of a di- or tricarboxylic acid, etc. The term "substantially equivalent" is used to mean that these activities are the same in nature (e.g., physiologically or pharmacologically). Accordingly, the transport of a di- or tricarboxylic acid is preferably equivalent (e.g., about 0.01- to about 100-fold, preferably about 0.1- to about 10-fold, more preferably about 0.5- to about 2-fold), but quantitative factors such as a level of these activities, a molecular weight of the protein, etc. may differ.

The activities such as the transport of a di- or tricarboxylic acid, etc. can be determined by a publicly known method with some modifications, for example, by the method described in, e.g., Am. J. Physiol. Cell. Physiol., 278, C1019-C1030 (2000) or its modifications.

The protein of the present invention includes muteins such as proteins containing, e.g., the following amino acid sequences: (1) (i) amino acid sequences represented by SEQ ID NO: 1, wherein at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are deleted; (ii) amino acid sequences represented by SEQ ID NO: 1, to which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are added; (iii) amino acid sequences represented by SEQ ID NO: 1, in which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are inserted; (iv) amino acid sequences represented by SEQ ID NO: 1, in which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are substituted by other amino acids; or (v) combination of these amino acid sequences described above; and (2) (i) amino acid sequences represented by SEQ ID NO: 23, wherein at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are deleted; (ii) amino acid sequences represented by SEQ ID NO: 23, to which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are added; (iii) amino acid sequences represented by SEQ ID NO: 23, in which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are inserted; (iv) amino acid sequences represented by SEQ ID NO: 23, in which at least 1 or 2 amino acids (e.g., approximately 1 to 200 amino acids, preferably approximately 1 to 50 amino acids, preferably approximately 1 to 30 amino acids, preferably approximately 1 to 10 amino acids, more preferably several (1 to 5) amino acids) are substituted by other amino acids; or (v) combination of these amino acid sequences described above; etc.

Where the amino acid sequences are inserted, deleted or substituted as described above, the position to be inserted, deleted or substituted is not particularly limited.

Throughout the present specification, the proteins are represented in accordance with the conventional way of describing peptides, that is, the N-terminus (amino terminus) at the left hand and the C-terminus (carboxyl terminus) at the right hand. In the proteins of the present invention including the proteins containing the amino acid sequence represented by SEQ ID NO: 1, the C-terminus is usually in the form of a carboxyl group (-COOH) or a carboxylate (-COO⁻) but may be in any form of an amide (-CONH₂) or an ester (-COOR).

Herein, examples of the ester group shown by R used include a C₁₋₆ alkyl group such as methyl, ethyl, n-propyl, isopropyl, n-butyl, etc.; a C₃₋₈ cycloalkyl group such as cyclopentyl, cyclohexyl, etc.; a C₆₋₁₂ aryl group such as phenyl, a-naphthyl, etc.; a C₇₋₁₄ aralkyl group such as a phenyl-C₁₋₂-alkyl group, e.g., benzyl, phenethyl, etc., or an a-naphthyl-C₁₋₂-alkyl group such as a-naphthylmethyl, etc.; pivaloyloxymethyl and the like.

Where the protein used in the present invention contains a carboxyl group (or a carboxylate) at a position other than the C-terminus, the carboxyl group may be amidated or esterified and such an amide or ester is also included within the protein used in the present invention. Examples of the ester group in this case include the same group as given with respect to the C-terminus described above.

Furthermore, the protein of the present invention includes variants of the above proteins, wherein the amino group at the N-terminal amino acid residue (e.g., methionine residue) in the protein of the present invention is protected with a protecting group (for example, a C₁₋₆ acyl group such as a C₁₋₆ alkanoyl group such as formyl group, acetyl group, etc.); those wherein the N-terminal glutamine residue is cleaved in vivo and the glutamyl group thus formed is pyroglutaminated; those wherein a substituent (e.g., -OH, -SH, amino group, imidazole group, indole group, guanidino group, etc.) on the side chain of an amino acid in the molecule is protected with a suitable protecting group (e.g., a C₁₋₆ acyl group such as a C₁₋₆ alkanoyl group, e.g., formyl group, acetyl group, etc.), or conjugated proteins such as glycoproteins bound to sugar chains.

Specific examples of the protein of the present invention include a human liver-derived protein containing the amino acid sequence represented by SEQ ID NO: 1, a mouse-derived protein containing the amino acid sequence represented by SEQ ID NO: 23, etc.

As partial peptides of the protein of the present invention, any partial peptide can be used so long as it is a partial peptide of the protein, preferably having the same property as that of the protein of the invention described above.

For example, peptides used are those having at least 20, preferably at least 50, more preferably at least 70, much more preferably at least 100, and most preferably at least 200 amino acids, in the amino acid sequence which constitutes the protein of the present invention.

The partial peptide of the present invention may contain an amino acid sequence, wherein at least 1 or 2 amino acids (preferably approximately 1 to 10 amino acids, more preferably several (1 to 5) amino acids) are deleted; to which at least 1 or 2 amino acids (preferably approximately 1 to 20 amino acids, more preferably approximately 1 to 10 amino acids, and most preferably several (1 to 5) amino acids) are added; wherein at least 1 or 2 amino acids (preferably approximately 1 to 10 amino acids, more preferably several (1 to 5) amino acids) are inserted; or, wherein at least 1 or 2 amino acids (preferably approximately 1 to 10 amino acids, more preferably several and most preferably approximately 1 to 5 amino acids) are substituted by other amino acids.

Furthermore, in the partial peptide of the present invention, the C-terminus may be in any form of a carboxyl group (-COOH), a carboxylate (-COO⁻), an amide (-CONH₂) or an ester (-COOR).

The partial peptides of the present invention further include those having a carboxyl group (or a carboxylate) at a position other than the C-terminus, those having an amino group in the N-terminal amino acid residue (e.g., methionine residue), which is protected with a protecting group, those wherein the amino group of the N-terminal amino acid residue (e.g., methionine residue) is protected with a protecting group, those in which the N-terminal residue is cleaved in vivo and the produced glutamine residue is pyroglutaminated, those in which substituents on the side chains of amino acids in the molecule are protected by appropriate protecting groups, conjugated peptides such as so-called glycoproteins, to which sugar chains are bound, and the like.

The partial peptides of the present invention can also be used as antigens for producing the antibodies. The partial peptides for producing the antibodies of the present invention include, for example, a peptide containing an amino acid sequence of 136-204, 228-252 or 276-309 in the amino acid sequence represented by SEQ ID NO: 1, a peptide containing an amino acid sequence of 136-207, 231-255 or 279-313 in the amino acid sequence represented by SEQ ID NO: 1, and the like.

For salts of the protein or the partial peptide of the present invention, preferred are salts with physiologically acceptable acids, especially physiologically acceptable acid addition salts. Examples of the salts include salts with, for example, inorganic acids (e.g., hydrochloric acid, phosphoric acid, hydrobromic acid, sulfuric acid); salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid) and the like.

The protein of the present invention, its partial peptides, or salts thereof may be manufactured by a publicly known method used to purify a protein from human and other warm-blooded animal cells or tissues described above, or by culturing a transformant that contains the DNA encoding the protein of the present invention, as will be later described. Furthermore, the protein or its salts may also be manufactured by the methods for synthesizing proteins or by modifications thereof, which will also be described hereinafter.

Where the protein of the present invention, its partial peptides, or salts thereof are manufactured from human and other mammalian tissues or cells, human and other mammalian tissues or cells are homogenized, then extracted with an acid or the like, and the extract is isolated and purified by a combination of chromatography techniques such as reverse phase chromatography, ion exchange chromatography, and the like.

To synthesize the protein of the present invention, its partial peptides, or salts or amides thereof, commercially available resins that are used for protein synthesis may be used. Examples of such resins include chloromethyl resin, hydroxymethyl resin, benzhydrylamine resin, aminomethyl resin, 4-benzyloxybenzyl alcohol resin, 4-methylbenzhydrylamine resin, PAM resin, 4-hydroxymethylmehtylphenyl acetamidomethyl resin, polyacrylamide resin, 4-(2',4'-dimethoxyphenylhydroxymethyl)phenoxy resin, 4-(2',4'-dimethoxyphenyl-Fmoc-aminoethyl) phenoxy resin, etc. Using these resins, amino acids in which α-amino groups and functional groups on the side chains are appropriately protected are condensed on the resin in the order of the sequence of the objective protein in accordance with various condensation methods publicly known in the art. At the end of the reaction, the protein or its partial peptides are cut out from the resin and at the same time, the protecting groups are removed therefrom. Then, intramolecular disulfide bond-forming reaction is performed in a highly diluted solution to obtain the objective protein or partial peptide, or amides thereof.

For condensation of the protected amino acids described above, a variety of activation reagents for protein synthesis may be used, and carbodiimides are particularly preferable. Examples of such carbodiimides include DCC, N,N'-diisopropylcarbodiimide, N-ethyl-N'-(3-dimethylaminoprolyl)carbodiimide, etc. For activation by these reagents, the protected amino acids in combination with a racemization inhibitor (e.g., HOBt, HOOBt) are added directly to the resin, or the protected amino acids are previously activated in the form of symmetric acid anhydrides, HOBt esters or HOOBt esters, followed by adding the thus activated protected amino acids to the resin.

Solvents suitable for use to activate the protected amino acids or condense with the resin may be chosen from solvents known to be usable for protein condensation reactions. Examples of such solvents are acid amides such as N,N-dimethylformamide, N,N-dimethylacetamide, N-methylpyrrolidone, etc.; halogenated hydrocarbons such as methylene chloride, chloroform, etc.; alcohols such as trifluoroethanol, etc.; sulfoxides such as dimethylsulfoxide, etc.; ethers such as pyridine, dioxane, tetrahydrofuran, etc.; nitriles such as acetonitrile, propionitrile, etc.; esters such as methyl acetate, ethyl acetate, etc.; and appropriate mixtures of these solvents. The reaction temperature is appropriately chosen from the range known to be applicable to protein binding reactions and is usually selected in the range of approximately -20°C to 50°C. The activated amino acid derivatives are used generally in an excess of 1.5 to 4 times. The condensation is examined by a test using the ninhydrin reaction; when the condensation is insufficient, the condensation can be completed by repeating the condensation reaction without removal of the protecting groups. When the condensation is yet insufficient even after repeating the reaction, unreacted amino acids are acetylated with acetic anhydride or acetylimidazole.

Examples of the protecting groups used to protect the amino groups of the starting compounds include Z, Boc, t-pentyloxycarbonyl, isobornyloxycarbonyl, 4-methoxybenzyloxycarbonyl, Cl-Z, Br-Z, adamantyloxycarbonyl, trifluoroacetyl, phthaloyl, formyl, 2-nitrophenylsulphenyl, diphenylphosphinothioyl, Fmoc, etc.

A carboxyl group can be protected by, e.g., alkyl esterification (in the form of linear, branched or cyclic alkyl esters of the alkyl moiety such as methyl, ethyl, propyl, butyl, t-butyl, cyclopentyl, cyclohexyl, cycloheptyl, cyclooctyl, 2-adamantyl, etc.), aralkyl esterification (e.g., esterification in the form of benzyl ester, 4-nitrobenzyl ester, 4-methoxybenzyl ester, 4-chlorobenzyl ester, benzhydryl ester, etc.), phenacyl esterification, benzyloxycarbonyl hydrazidation, t-butoxycarbonyl hydrazidation, trityl hydrazidation, or the like.

The hydroxyl group of serine can be protected through, for example, its esterification or etherification. Examples of groups appropriately used for the esterification include a lower (C₁₋₆) alkanoyl group, such as acetyl group, an aroyl group such as benzoyl group, and a group derived from carbonic acid such as benzyloxycarbonyl group, ethoxycarbonyl group, etc. Examples of a group appropriately used for the etherification include benzyl group, tetrahydropyranyl group, t-butyl group, etc.

Examples of groups for protecting the phenolic hydroxyl group of tyrosine include Bzl, Cl₂-Bzl, 2-nitrobenzyl, Br-Z, t-butyl, etc.

Examples of groups used to protect the imidazole moiety of histidine include Tos, 4-methoxy-2,3,6-trimethylbenzenesulfonyl, DNP, benzyloxymethyl, Bum, Boc, Trt, Fmoc, etc.

Examples of the activated carboxyl groups in the starting compounds include the corresponding acid anhydrides, azides, activated esters [esters with alcohols (e.g., pentachlorophenol, 2,4,5-trichlorophenol, 2,4-dinitrophenol, cyanomethyl alcohol, p-nitrophenol, HONB, N-hydroxysuccimide, N-hydroxyphthalimide, HOBt)], etc. As the activated amino acids, in which the amino groups are activated in the starting material, for example, the corresponding phosphoric amides are employed.

To eliminate (split off) the protecting groups, there are used catalytic reduction under hydrogen gas flow in the presence of a catalyst such as Pd-black or Pd-carbon; an acid treatment with anhydrous hydrogen fluoride, methanesulfonic acid, trifluoromethane-sulfonic acid or trifluoroacetic acid, or a mixture solution of these acids; a treatment with a base such as diisopropylethylamine, triethylamine, piperidine or piperazine; and reduction with sodium in liquid ammonia. The elimination of the protecting group by the acid treatment described above is carried out generally at a temperature of approximately -20°C to 40°C. In the acid treatment, it is efficient to add a cation scavenger such as anisole, phenol, thioanisole, m-cresol, p-cresol, dimethylsulfide, 1,4-butanedithiol or 1,2-ethanedithiol. Furthermore, 2,4-dinitrophenyl group known as the protecting group for the imidazole of histidine is removed by a treatment with thiophenol. Formyl group used as the protecting group of the indole of tryptophan is eliminated by the aforesaid acid treatment in the presence of 1,2-ethanedithiol, 1,4-butanedithiol or the like, as well as by a treatment with an alkali such as a dilute sodium hydroxide solution, dilute ammonia, etc.

Protection of functional groups that should not be involved in the reaction of the starting materials, protecting groups, elimination of the protecting groups and activation of functional groups involved in the reaction may be appropriately selected from publicly known groups and publicly known means.

In another method for obtaining the amides of the protein or its partial peptides, for example, the α-carboxyl group of the carboxy terminal amino acid is first protected by amidation; the peptide (protein) chain is then extended from the amino group side to a desired length. Thereafter, a protein or its partial peptides, from which only the protecting group of the N-terminal α-amino group in the peptide chain has been eliminated, and a protein or its partial peptides, from which only the protecting group of the C-terminal carboxyl group has been eliminated are prepared. These proteins or partial peptides are condensed in a mixture of the solvents described above. The details of the condensation reaction are the same as described above. After the protected protein or peptide obtained by the condensation is purified, all the protecting groups are eliminated by the method described above to give the desired crude protein or peptide. This crude protein or peptide is purified by various known purification means. Lyophilization of the major fraction gives the amide of the desired protein or peptide.

To prepare the esterified protein or peptide, for example, the α-carboxyl group of the carboxy terminal amino acid is condensed with a desired alcohol to prepare the amino acid ester, which is followed by procedure similar to the preparation of the amidated protein or peptide above to give the ester form of the desired protein or peptide.

The partial peptide or its salts used in the present invention can be manufactured by publicly known methods for peptide synthesis, or by cleaving the protein of the present invention with an appropriate peptidase. For the methods for peptide synthesis, for example, either solid phase synthesis or liquid phase synthesis may be used. That is, the partial peptide or amino acids that can construct the partial peptide used in the present invention are condensed with the remaining part.

Where the product contains protecting groups, these protecting groups are removed to give the desired peptide. Publicly known methods for condensation and elimination of the protecting groups are described in (1) - (5) below.
(1) M. Bodanszky & M.A. Ondetti: Peptide Synthesis, Interscience Publishers, New York (1966)
(2) Schroeder & Luebke: The Peptide, Academic Press, New York (1965)
(3) Nobuo Izumiya, et al.: *Peptide Gosei-no-Kiso to Jikken* (Basics and experiments of peptide synthesis), published by Maruzen Co. (1975)
(4) Haruaki Yajima & Shunpei Sakakibara: *Seikagaku Jikken Koza* (Biochemical Experiment) 1, *Tanpakushitsu no Kagaku* (Chemistry of Proteins) IV, 205 (1977)
(5) Haruaki Yajima, ed.: *Zoku lyakuhin no Kaihatsu* (A sequel to Development of Pharmaceuticals), Vol. 14, Peptide Synthesis, published by Hirokawa Shoten

After completion of the reaction, the produce may be purified and isolated by a combination of conventional purification methods such as solvent extraction, distillation, column chromatography, liquid chromatography, recrystallization and the like to give the partial peptide used in the present invention. When the partial peptide obtained by the above methods is in a free form, the peptide can be converted into an appropriate salt by a publicly known method; when the partial peptide is obtained in a salt form, it can be converted into a free form or in the form of a different salt by a publicly known method or its modifications.

The polynucleotide encoding the protein used in the present invention may be any polynucleotide so long as it contains the base sequence encoding the protein used in the present invention described above. Preferably, the polynucleotide is DNA. Such DNA may be any one of genomic DNA, genomic DNA library, cDNA derived from the cells/tissues described above, cDNA library derived from the cells/tissues described above and synthetic DNA.

The vector to be used for the library may be any of bacteriophage, plasmid, cosmid, phagemid, ane the like. The DNA may also be directly amplified by reverse transcriptase polymerase chain reaction (hereinafter abbreviated as RT-PCR) using the total RNA or mRNA fraction prepared from the cells/tissues described above.

The DNA encoding the protein used in the present invention may be any DNA such as (i) DNA containing the base sequence represented by SEQ ID NO: 2, or DNA containing a base sequence hybridizable to the base sequence represented by SEQ ID NO: 2 under highly stringent conditions and encoding a protein having the activities substantially equivalent to those of the protein containing the amino acid sequence represented by SEQ ID NO: 1, (ii) DNA containing the base sequence represented by SEQ ID NO: 22, or DNA containing a base sequence hybridizable to the base sequence represented by SEQ ID NO: 22 under highly stringent conditions and encoding a protein having the activities substantially equivalent to those of the protein containing the amino acid sequence represented by SEQ ID NO: 1, (iii) DNA containing the base sequence represented by SEQ ID NO: 24, or DNA containing a base sequence hybridizable to the base sequence represented by SEQ ID NO: 24 under highly stringent conditions and encoding a protein having the activities substantially equivalent to those of the protein containing the amino acid sequence represented by SEQ ID NO: 23, and (iv) DNA containing the base sequence represented by SEQ ID NO: 36, or DNA containing a base sequence hybridizable to the base sequence represented by SEQ ID NO: 36 under highly stringent conditions and encoding a protein having the activities substantially equivalent to those of the protein containing the amino acid sequence represented by SEQ ID NO: 23.

Examples of the DNA hybridizable to the base sequence represented by SEQ ID NO: 2 or SEQ ID NO: 22 under highly stringent conditions include DNA containing a base sequence having at least about 60%, preferably at least about 70% homology, preferably at least about 80% homology, and most preferably at least about 95 % homology, to the base sequence represented by SEQ ID NO: 2 or SEQ ID NO: 22.

Examples of the DNA hybridizable to the base sequence represented by SEQ ID NO: 24 or SEQ ID NO: 36 under highly stringent conditions include DNA containing a base sequence having at least about 50%, preferably at least about 60%, preferably at least about 70% homology, preferably at least about 80% homology, and most preferably at least about 95% homology, to the base sequence represented by SEQ ID NO: 24 or SEQ ID NO: 36.

The hybridization can be carried out by publicly known methods or by modifications of these methods, for example, according to the method described in Molecular Cloning, 2nd (J. Sambrook et al., Cold Spring Harbor Lab. Press, 1989). A commercially available library may also be used according to the instructions of the attached manufacturer's protocol. Preferably, the hybridization can be carried out under highly stringent conditions.

The highly stringent conditions used herein are, for example, those in a sodium concentration at approximately 19 to 40 mM, preferably approximately 19 to 20 mM at a temperature of approximately 50 to 70°C, preferably approximately 60 to 65°C. In particular, hybridization conditions in a sodium concentration of about 19 mM at a temperature of about 65°C are most preferred.

The polynucleotide encoding the partial peptide of the present invention may be any polynucleotide so long as it contains the base sequence encoding the partial peptide of the present invention described above. Preferably, the polynucleotide is DNA. Such DNA may be any one of genomic DNA, genomic DNA library, cDNA derived from the cells/tissues described above, cDNA library derived from the cells/tissues described above and synthetic DNA.

The polynucleotide encoding the partial peptide of the present invention may be any polynucleotide such as (1) a polynucleotide containing a part of polynucleotide containing the base sequence represented by SEQ ID NO: 2 or SEQ ID NO: 22, (2) a polynucleotide containing a part of polynucleotide containing a base sequence hybridizable to the base sequence represented by SEQ ID NO: 2 or SEQ ID NO: 22 under highly stringent conditions and encoding a protein having the activities substantially equivalent to those of the protein containing the amino acid sequence represented by SEQ ID NO: 1, (3) a polynucleotide containing a part of polynucleotide containing the base sequence represented by SEQ ID NO: 24 or SEQ ID NO: 36, or (4) a polynucleotide containing a part of polynucleotide containing a base sequence hybridizable to the base sequence represented by SEQ ID NO: 24 or SEQ ID NO: 36 under highly stringent conditions and encoding a protein having the activities substantially equivalent to those of the protein containing the amino acid sequence represented by SEQ ID NO: 23, etc.

The polynucleotide hybridizable to the base sequence represented by SEQ ID NO: 2 or SEQ ID NO: 22 and the polynucleotide hybridizable to the base sequence represented by SEQ ID NO: 22 or SEQ ID NO: 36 are the same as describe above.

Methods for hybridization and highly stringent conditions used are the same as described above.

For cloning of the DNA that completely encodes the protein of the present invention or its partial peptide (hereinafter sometimes collectively referred to as the protein of the present invention in the section describing the cloning of DNA and expression), the DNA may be either amplified by PCR using synthetic DNA primers containing a part of the base sequence encoding the protein of the present invention, or the DNA inserted into an appropriate vector can be selected by hybridization with a labeled DNA fragment or synthetic DNA that encodes a part or entire region of the protein of the present invention. The hybridization can be carried out, for example, according to the method described in Molecular Cloning, 2nd. (J. Sambrook et al., Cold Spring Harbor Lab. Press, 1989). The hybridization may also be performed using commercially available library in accordance with the protocol described in the attached instructions.

Conversion of the base sequence of the DNA can be effected by publicly known methods such as the ODA-LA PCR method, the Gapped duplex method or the Kunkel method or its modification by using a publicly known kit available as Mutan™ -super Express Km or Mutan™ -K (both manufactured by Takara Shuzo Co., Ltd.), etc.

The cloned DNA encoding the protein of the present invention can be used as it is, depending upon purpose or, if desired, after digestion with a restriction enzyme or after addition of a linker thereto. The DNA may contain ATG as a translation initiation codon at the 5' end thereof and may further contain TAA, TGA or TAG as a translation termination codon at the 3' end thereof. These translation initiation and termination codons may also be added by using an appropriate synthetic DNA adapter.

The expression vector for the protein of the present invention can be manufactured, for example, by (a) excising the desired DNA fragment from the DNA, e.g., cDNA, encoding the protein of the present invention, and then (b) ligating the DNA fragment with an appropriate expression vector downstream a promoter in the expression vector.

Examples of the vector include plasmids derived form E. coli (e.g., pBR322, pBR325, pUC12, pUC13), plasmids derived from Bacillus subtilis (e.g., pUB110, pTP5, pC194), plasmids derived from yeast (e.g., pSH19, pSH15), bacteriophages such as ? phage, etc., animal viruses such as retrovirus, vaccinia virus, baculovirus, etc. as well as pA 1-11, pXT1, pRc/CMV, pRc/RSV, pcDNAI/Neo, etc.

The promoter used in the present invention may be any promoter if it matches well with a host to be used for gene expression. In the case of using animal cells as the host, examples of the promoter include SRa promoter, SV40 promoter, LTR promoter, CMV promoter, HSV-TK promoter, etc.

Among them, CMV (cytomegalovirus) promoter or SRα promoter is preferably used. Where the host is bacteria of the genus Escherichia, preferred examples of the promoter include trp promoter, lac promoter, recA promoter, ?P_{L} promoter, lpp promoter, T7 promoter, etc. In the case of using bacteria of the genus Bacillus as the host, preferred example of the promoter are SPO1 promoter, SPO2 promoter, penP promoter, etc. When yeast is used as the host, preferred examples of the promoter are PHO5 promoter, PGK promoter, GAP promoter, ADH promoter, etc. When insect cells are used as the host, preferred examples of the promoter include polyhedrin prompter, P10 promoter, etc.

In addition to the foregoing examples, the expression vector may further optionally contain an enhancer, a splicing signal, a polyA addition signal, a selection marker, SV40 replication origin (hereinafter sometimes abbreviated as SV40ori) etc. Examples of the selection marker include dihydrofolate reductase (hereinafter sometimes abbreviated as dhfr) gene [methotrexate (MTX) resistance], ampicillin resistant gene (hereinafter sometimes abbreviated as Amp^{r}), neomycin resistant gene (hereinafter sometimes abbreviated as Neo^{r}, G418 resistance), etc. In particular, when dhfr gene is used as the selection marker in dhfr gene-deficient Chinese hamster cells, selection can also be made on thymidine free media.

If necessary, a signal sequence that matches with a host is added to the N-terminus of the protein of the present invention. Examples of the signal sequence that can be used are Pho A signal sequence, OmpA signal sequence, etc. in the case of using bacteria of the genus Escherichia as the host; a-amylase signal sequence, subtilisin signal sequence, etc. in the case of using bacteria of the genus Bacillus as the host; MFa signal sequence, SUC2 signal sequence, etc. in the case of using yeast as the host; and insulin signal sequence, a-interferon signal sequence, antibody molecule signal sequence, etc. in the case of using animal cells as the host, respectively.

Using the vector containing the DNA encoding the protein of the present invention thus constructed, transformants can be manufactured.

Examples of the host, which may be employed, are bacteria belonging to the genus Escherichia, bacteria belonging to the genus Bacillus, yeast, insect cells, insects, animal cells, etc.

Specific examples of the bacteria belonging to the genus Escherichia include Escherichia coli K12 DH1 [Proc. Natl. Acad. Sci. U.S.A., 60, 160 (1968)], JM103 (Nucleic Acids Research, 9, 309 (1981)], JA221 [Journal of Molecular Biology, 120, 517 (1978)], HB101 [Journal of Molecular Biology, 41, 459 (1969)], C600 [Genetics, 39, 440 (1954)], etc.

Examples of the bacteria belonging to the genus Bacillus include Bacillus subtilis MI114 [Gene, 24, 255 (1983)], 207-21 [Journal of Biochemistry, 95, 87 (1984)], etc.

Examples of yeast include Saccharomyces cereviseae AH22, AH22R⁻, NA87-11A, DKD-5D, 20B-12, Schizosaccharomyces pombe NCYC1913, NCYC2036, Pichia pastoris KM71, etc.

Examples of insect cells include, for the virus AcNPV, Spodoptera frugiperda cells (Sf cells), MG1 cells derived from mid-intestine of Trichoplusia ni, High Five™ cells derived from egg of Trichoplusia ni, cells derived from Mamestra brassicae, cells derived from Estigmena acrea, etc.; and for the virus BmNPV, Bombyx mori N cells (BmN cells), etc. are used. Examples of the Sf cell which can be used are Sf9 cells (ATCC CRL1711) and Sf21 cells (both cells are described in Vaughn, J. L. et al., In Vivo, 13, 213-217 (1977)), etc.

As the insect, for example, a larva of Bombyx mori can be used [Maeda, et al., Nature, 315, 592 (1985)], etc.

Examples of animal cells include monkey cells COS-7, Vero, Chinese hamster cells CHO (hereinafter abbreviated as CHO cells), dhfr gene deficient Chinese hamster cells CHO (hereinafter abbreviated as CHO (dhfr⁻) cell), mouse L cells, mouse AtT-20, mouse myeloma cells, rat GH3, human FL cells, etc.

Bacteria belonging to the genus Escherichia can be transformed, for example, by the method described in Proc. Natl. Acad. Sci. U.S.A., 69, 2110 (1972) or Gene, 17, 107 (1982), etc.

Bacteria belonging to the genus Bacillus can be transformed, for example, by the method described in Molecular & General Genetics, 168, 111 (1979), etc.

Yeast can be transformed, for example, by the method described in Methods in Enzymology, 194, 182-187 (1991), Proc. Natl. Acad. Sci. U.S.A., 75, 1929 (1978), etc.

Insect cells or insects can be transformed, for example, according to the method described in Bio/Technology, 6, 47-55(1988), etc.

Animal cells can be transformed, for example, according to the method described in *Saibo Kogaku* (Cell Engineering), extra issue 8, *Shin Saibo Kogaku Jikken Protocol* (New Cell Engineering Experimental Protocol), 263-267 (1995), published by Shujunsha, or Virology, 52, 456 (1973).

Thus, the transformant transformed with the expression vector containing the DNA encoding the protein can be obtained.

Where the host is bacteria belonging to the genus Escherichia or the genus Bacillus, the transformant can be appropriately incubated in a liquid medium which contains materials required for growth of the transformant such as carbon sources, nitrogen sources, inorganic materials, and so on. Examples of the carbon sources include glucose, dextrin, soluble starch, sucrose, etc. Examples of the nitrogen sources include inorganic or organic materials such as ammonium salts, nitrate salts, corn steep liquor, peptone, casein, meat extract, soybean cake, potato extract, etc. Examples of the inorganic materials are calcium chloride, sodium dihydrogenphosphate, magnesium chloride, etc. In addition, yeast extract, vitamins, growth promoting factors etc. may also be added to the medium. Preferably, pH of the medium is adjusted to about 5 to about 8.

A preferred example of the medium for incubation of the bacteria belonging to the genus Escherichia is M9 medium supplemented with glucose and Casamino acids [Miller, Journal of Experiments in Molecular Genetics, 431-433, Cold Spring Harbor Laboratory, New York, 1972]. If necessary, a chemical such as 3β-indolylacrylic acid can be added to the medium thereby to activate the promoter efficiently.

Where the bacteria belonging to the genus Escherichia are used as the host, the transformant is usually cultivated at about 15°C to about 43°C for about 3 hours to about 24 hours. If necessary, the culture may be aerated or agitated.

Where the bacteria belonging to the genus Bacillus are used as the host, the transformant is cultivated generally at about 30°C to about 40°C for about 6 hours to about 24 hours. If necessary, the culture can be aerated or agitated.

Where yeast is used as the host, the transformant is cultivated, for example, in Burkholder's minimal medium [Bostian, K. L. et al., Proc. Natl. Acad. Sci. U.S.A., 77, 4505 (1980)] or in SD medium supplemented with 0.5% Casamino acids [Bitter, G. A. et al., Proc. Natl. Acad. Sci. U.S.A., 81, 5330 (1984)]. Preferably, pH of the medium is adjusted to about 5 to about 8. In general, the transformant is cultivated at about 20°C to about 35°C for about 24 hours to about 72 hours. If necessary, the culture can be aerated or agitated.

Where insect cells or insects are used as the host, the transformant is cultivated in, for example, Grace's Insect Medium (Grace, T. C. C., Nature, 195, 788 (1962)) to which an appropriate additive such as immobilized 10% bovine serum is added. Preferably, pH of the medium is adjusted to about 6.2 to about 6.4. Normally, the transformant is cultivated at about 27°C for about 3 days to about 5 days and, if necessary and desired, the culture can be aerated or agitated.

Where animal cells are employed as the host, the transformant is cultivated in, for example, MEM medium containing about 5% to about 20% fetal calf serum [Science, 122, 501 (1952)], DMEM medium [Virology, 8, 396 (1959)], RPMI 1640 medium [The Journal of the American Medical Association, 199, 519 (1967)], 199 medium [Proceeding of the Society for the Biological Medicine, 73, 1 (1950)], etc. Preferably, pH of the medium is adjusted to about 6 to about 8. The transformant is usually cultivated at about 30°C to about 40°C for about 15 hours to about 60 hours and, if necessary and desired, the culture can be aerated or agitated.

As described above, the protein of the present invention can be produced into the cell, on the cell membrane or out of the cell of the transformant.

The protein of the present invention can be separated and purified from the culture described above by the following procedures.

When the protein of the present invention is extracted from the culture or cells, after cultivation the transformants or cells are collected by a publicly known method and suspended in an appropriate buffer. The transformants or cells are then disrupted by publicly known methods such as ultrasonication, a treatment with lysozyme and/or freeze-thaw cycling, followed by centrifugation, filtration, etc. Thus, the crude extract of the protein of the present invention can be obtained. The buffer used for the procedures may contain a protein modifier such as urea or guanidine hydrochloride, or a surfactant such as Triton X-100™ , etc. When the protein is secreted in the culture, after completion of the cultivation the supernatant can be separated from the transformants or cells to collect the supernatant by a publicly known method.

The protein contained in the supernatant or the extract thus obtained can be purified by appropriately combining the publicly known methods for separation and purification. Such publicly known methods for separation and purification include a method utilizing difference in solubility such as salting out, solvent precipitation, etc.; a method utilizing mainly difference in molecular weight such as dialysis, ultrafiltration, gel filtration, SDS-polyacrylamide gel electrophoresis, etc.; a method utilizing difference in electric charge such as ion exchange chromatography, etc.; a method utilizing difference in specific affinity such as affinity chromatography, etc.; a method utilizing difference in hydrophobicity such as reverse phase high performance liquid chromatography, etc.; a method utilizing difference in isoelectric point such as isoelectrofocusing electrophoresis; and the like.

When the protein thus obtained is in a free form, it can be converted into the salt by publicly known methods or modifications thereof. On the other hand, when the protein is obtained in the form of a salt, it can be converted into the free form or in the form of a different salt by publicly known methods or modifications thereof.

The protein produced by the recombinant can be treated, prior to or after the purification, with an appropriate protein modifying enzyme so that the protein can be appropriately modified to partially remove a polypeptide. Examples of the protein-modifying enzyme include trypsin, chymotrypsin, arginyl endopeptidase, protein kinase, glycosidase or the like.

The presence of the thus produced protein of the present invention thereof can be determined by an enzyme immunoassay using a specific antibody, or by western blotting, etc.

Antibodies to the protein of the present invention, its partial peptides, or salts thereof may be any of polyclonal antibodies and monoclonal antibodies, as long as they are capable of recognizing the protein of the present invention, its partial peptides, or salts thereof.

The antibodies to the protein of the present invention, its partial peptides, or salts thereof (hereinafter sometimes merely referred to as the protein of the present invention in the section describing the antibodies) may be manufactured by publicly known methods for manufacturing antibodies or antisera, using as antigens the protein of the present invention.

### [Preparation of monoclonal antibody]

### (a) Preparation of monoclonal antibody-producing cells

The protein of the present invention is administered to warm-blooded animals either solely or together with carriers or diluents to the site where the production of antibody is possible by the administration. In order to potentiate the antibody productivity upon the administration, complete Freund's adjuvants or incomplete Freund's adjuvants may be administered. The administration is usually carried out once in every 2 to 6 weeks and approximately 2 to 10 times in total. Examples of the applicable warm-blooded animals are monkeys, rabbits, dogs, guinea pigs, mice, rats, sheep, goats and chicken, with mice and rats being preferred.

In the preparation of monoclonal antibody-producing cells, warm-blooded animals, e.g., mice, immunized with an antigen wherein the antibody titer is noted is selected, then the spleen or lymph node is collected after 2 to 5 days from the final immunization and antibody-producing cells contained therein are fused with myeloma cells to give monoclonal antibody-producing hybridomas. Measurement of the antibody titer in antisera may be made, for example, by reacting a labeled form of the protein, which will be described later, with the antiserum followed by assaying the binding activity of the labeling agent bound to the antibody. The fusion may be operated, for example, by the known Koehler and Milstein method [Nature, 256, 495 (1975)]. Examples of the fusion accelerator are polyethylene glycol (PEG), Sendai virus, etc., of which PEG is preferably employed.

Examples of the myeloma cells are NS-1, P3U1, SP2/0, AP-1, etc. In particular, P3U1 is preferably employed. A preferred ratio of the count of the antibody-producing cells used (spleen cells) to the count of myeloma cells is within a range of approximately 1:1 to 20:1. When PEG (preferably, PEG 1000 to PEG 6000) is added in a concentration of approximately 10 to 80% followed by incubating at 20 to 40°C, preferably at 30 to 37°C for 1 to 10 minutes, an efficient cell fusion can be carried out.

Various methods can be used for screening of a monoclonal antibody-producing hybridoma. Examples of such methods include a method which comprises adding the supernatant of a hybridoma to a solid phase (e.g., microplate) adsorbed with the protein etc. as an antigen directly or together with a carrier, adding an anti-immunoglobulin antibody (when mouse cells are used for the cell fusion, anti-mouse immunoglobulin antibody is used) labeled with a radioactive substance or an enzyme, or Protein A and detecting the monoclonal antibody bound to the solid phase; a method which comprises adding the supernatant of a hybridoma to a solid phase adsorbed with an anti-immunoglobulin antibody or Protein A, adding the protein labeled with a radioactive substance or an enzyme and detecting the monoclonal antibody bound to the solid phase; and the like.

The monoclonal antibody can be selected by publicly known methods or by modifications of these methods. In general, the selection can be effected in a medium for animal cells supplemented with HAT (hypoxanthine, aminopterin and thymidine). Any selection and growth medium can be employed as far as the hybridoma can grow therein. For example, RPMI 1640 medium containing 1% to 20%, preferably 10% to 20% fetal calf serum, GIT medium (Wako Pure Chemical Industries, Ltd.) containing 1% to 10% fetal calf serum, a serum free medium for incubation of a hybridoma (SFM-101, Nissui Seiyaku Co., Ltd.) and the like may be used for the selection and growth medium. The incubation is carried out generally at 20°C to 40°C, preferably at about 37°C, for 5 days to 3 weeks, preferably 1 to 2 weeks. The incubation may be carried out normally in 5% CO₂. The antibody titer of the culture supernatant of hybridomas can be determined as in the assay for the antibody titer in the antisera described above.

### (b) Purification of monoclonal antibody

Separation and purification of the monoclonal antibody can be carried out by methods applied to conventional separation and purification of immunoglobulins, as in the conventional methods for separation and purification of polyclonal antibodies [e.g., salting-out, alcohol precipitation, isoelectric point precipitation, electrophoresis, adsorption and desorption with ion exchangers (e.g., DEAE), ultracentrifugation, gel filtration, or a specific purification method which comprises collecting only an antibody with an activated adsorbent such as an antigen-binding solid phase, Protein A, Protein G, etc. and dissociating the binding to obtain the antibody].

### [Preparation of polyclonal antibody]

The polyclonal antibody of the present invention can be manufactured by publicly known methods or modifications thereof. For example, an immunogen (protein antigen) itself, or a complex of the protein antigen and a carrier protein is prepared, and a warm-blooded animal is immunized with the immunogen or complex in a manner similar to the method described above for the manufacture of monoclonal antibodies. The product containing the antibody to the protein of the present invention is collected from the immunized animal followed by separation and purification of the antibody.

In the complex of an immunogen and a carrier protein used to immunize a warm-blooded animal, the type of carrier protein and the mixing ratio of the carrier to hapten may be of any type in any ratio, as long as the antibody is efficiently produced to the hapten immunized by crosslinking to the carrier. For example, bovine serum albumin, bovine thyroglobulins, keyhole limpet hemocyanin, etc. is coupled to hapten in a carrier-to-hapten weight ratio of approximately 0.1 to 20, preferably about 1 to about 5.

A variety of condensing agents can be used for the coupling of a carrier to hapten. Glutaraldehyde, carbodiimide, maleimide activated ester, activated ester reagents containing thiol group or dithiopyridyl group, etc. are used for the coupling.

The condensation product is administered to a warm-blooded animal either solely or together with carriers or diluents to the site in which the antibody can be produced by the administration. In order to potentiate the antibody productivity upon the administration, complete Freund's adjuvant or incomplete Freund's adjuvant may be administered. The administration is usually made once approximately in approximately every 2 to 6 weeks and about 3 to about 10 times in total.

The polyclonal antibody can be collected from the blood, ascites, etc., preferably from the blood, of warm-blooded animal immunized by the method described above.

The polyclonal antibody titer in antiserum can be assayed by the same procedure as that for the determination of serum antibody titer described above. The separation and purification of the polyclonal antibody can be carried out, following the method for the separation and purification of immunoglobulins performed as applied to the separation and purification of monoclonal antibodies described hereinabove.

The antisense polynucleotide containing the entire or part of a base sequence complementary or substantially complementary to the base sequence of DNA encoding the protein used in the present invention or its partial peptide (hereinafter these DNAs are sometimes referred to as the DNA of the present invention in the section describing the antisense polynucleotide) may be any antisense polynucleotide, so long as it contains the entire or part of a base sequence complementary or substantially complementary to the base sequence of the DNA of the present invention and is capable of suppressing expression of the DNA.

The base sequence substantially complementary to the DNA of the present invention may, for example, be a base sequence having at least about 70% homology, preferably at least about 80% homology, more preferably at least about 90% homology and most preferably at least about 95% homology, to the entire base sequence or partial base sequence of the base sequence complementary to the DNA of the present invention (i.e., complementary strand to the DNA of the present invention). Particularly in the entire base sequence of the complementary strand to the DNA of the present invention, an antisense polynucleotide having at least about 70% homology, preferably at least about 80% homology, more preferably at least about 90% homology and most preferably at least about 95% homology, to the complementary strand of the base sequence, which encodes the N-terminal region of the protein of the present invention, is preferred.

Specific examples include an antisense polynucleotide containing the entire or part of a base sequence complementary or substantially complementary to a base sequence of DNA containing the base sequence represented by SEQ ID NO: 2 or SEQ ID NO: 24, preferably an antisense polynucleotide containing the entire or part of a base sequence complementary to a base sequence of DNA containing the base sequence represented by SEQ ID NO: 2 or SEQ ID NO: 24, etc.

The antisense polynucleotide is generally constructed by bases of about 10 to about 40, preferably about 15 to about 30.

To prevent digestion with a hydrolase such as nuclease, etc., the phosphoric acid residue (phosphate) of each nucleotide that constructs the antisense DNA may be substituted with chemically modified phosphoric acid residues, e.g., phosphorothioate, methyl phosphonate, phosphorodithionate, etc. These antisense nucleotides may be synthesized using a publicly known DNA synthesizer, etc.

According to the present invention, the antisense polynucleotide (nucleic acid) corresponding to the protein gene of the present invention that can inhibit replication or expression of the protein genes of the present invention can be designed and synthesized based on the base sequence information of the cloned or determined DNA. Such a polynucleotide (nucleic acid) is hybridizable to RNA of the protein gene to inhibit the synthesis or function of said RNA or is capable of modulating or controlling the expression of a protein gene via interaction with RNA associated with the protein of the present invention. Polynucleotides complementary to the selected sequences of RNA associated with the protein of the present invention and polynucleotides specifically hybridizable to RNA associated with the protein of the present invention are useful in modulating or controlling the in vivo and in vitro expression of the protein gene of the present invention, and are useful for the treatment or diagnosis of diseases, etc. The term "corresponding" is used to mean homologous to or complementary to a particular sequence of the nucleotide, base sequence or nucleic acid including the gene. The term "corresponding" between nucleotides, base sequences or nucleic acids and peptides (proteins) usually refer to amino acids of a peptide (protein) under the order derived from the sequence of nucleotides (nucleic acids) or their complements. In the protein genes, the 5' end hairpin loop, 5' end 6-base-pair repeats, 5' end untranslated region, polypeptide translation initiation codon, protein coding region, ORF translation termination codon, 3' end untranslated region, 3' end palindrome region, and 3' end hairpin loop, may be selected as preferred target regions, though any other region may be selected as a target in the protein genes.

The relationship between the targeted nucleic acids and the polynucleotides complementary to at least a part of the target region, specifically the relationship between the target nucleic acids and the polynucleotides hybridizable to the target region, can be denoted to be "antisense" to the polynucleotides in the said target region. Examples of the antisense polynucleotides include polydeoxynucleotides containing 2-deoxy-D-ribose, polynucleotides containing D-ribose, any other type of polynucleotides which are N-glycosides of a purine or pyrimidine base, or other polymers containing non-nucleotide backbones (e.g., commercially available protein nucleic acids and synthetic sequence-specific nucleic acid polymers) or other polymers containing nonstandard linkages (provided that the polymers contain nucleotides having such a configuration that allows base pairing or base stacking, as is found in DNA or RNA), etc. The antisense polynucleotides may be double-stranded DNA, single-stranded DNA, double-stranded RNA, single-stranded RNA or a DNA:RNA hybrid, and may further include unmodified polynucleotides (or unmodified oligonucleotides), those with publicly known types of modifications, for example, those with labels known in the art, those with caps, methylated polynucleotides, those with substitution of one or more naturally occurring nucleotides by their analogue, those with intramolecular modifications of nucleotides such as those with uncharged linkages (e.g., methyl phosphonates, phosphotriesters, phosphoramidates, carbamates, etc.) and those with charged linkages or sulfur-containing linkages (e.g., phosphorothioates, phosphorodithioates, etc.), those having side chain groups such as proteins (nucleases, nuclease inhibitors, toxins, antibodies, signal peptides, poly-L-lysine, etc.), saccharides (e.g., monosaccharides, etc.), those with intercalators (e.g., acridine, psoralen, etc.), those containing chelators (e.g., metals, radioactive metals, boron, oxidative metals, etc.), those containing alkylating agents, those with modified linkages (e.g., a anomeric nucleic acids, etc.), and the like. Herein the terms "nucleoside", "nucleotide" and "nucleic acid" are used to refer to moieties that contain not only the purine and pyrimidine bases, but also other heterocyclic bases, which have been modified. Such modifications may include methylated purines and pyrimidines, acylated purines and pyrimidines and other heterocyclic rings. Modified nucleotides and modified nucleotides also include modifications on the sugar moiety, wherein, for example, one or more hydroxyl groups may optionally be substituted with a halogen atom(s), an aliphatic group(s), etc., or may be converted into the corresponding functional groups such as ethers, amines, or the like.

The antisense polynucleotide (nucleic acid) of the present invention is RNA, DNA or a modified nucleic acid (RNA, DNA). Specific examples of the modified nucleic acid are, but not limited to, sulfur and thiophosphate derivatives of nucleic acids and those resistant to degradation of polynucleoside amides or oligonucleoside amides. The antisense nucleic acids of the present invention can be modified preferably based on the following design, that is, by increasing the intracellular stability of the antisense nucleic acid, increasing the cellular permeability of the antisense nucleic acid, increasing the affinity of the nucleic acid to the targeted sense strand to a higher level, or minimizing the toxicity, if any, of the antisense nucleic acid.

Many of such modifications are known in the art, as disclosed in J. Kawakami, et al., Pharm. Tech. Japan, Vol. 8, pp. 247, 1992; Vol. 8, pp. 395, 1992; S. T. Crooke, et al. ed., Antisense Research and Applications, CRC Press, 1993; etc.

The antisense nucleic acid of the present invention may contain altered or modified sugars, bases or linkages. The antisense nucleic acid may also be provided in a specialized form such as liposomes, microspheres, or may be applied to gene therapy, or may be provided in combination with attached moieties. Such attached moieties include polycations such as polylysine that act as charge neutralizers of the phosphate backbone, or hydrophobic moieties such as lipids (e.g., phospholipids, cholesterols, etc.) that enhance the interaction with cell membranes or increase uptake of the nucleic acid. Preferred examples of the lipids to be attached are cholesterols or derivatives thereof (e.g., cholesteryl chloroformate, cholic acid, etc.). These moieties may be attached to the nucleic acid at the 3' or 5' ends thereof and may also be attached thereto through a base, sugar, or intramolecular nucleoside linkage. Other moieties may be capping groups specifically placed at the 3' or 5' ends of the nucleic acid to prevent degradation by nucleases such as exonuclease, RNase, etc. Such capping groups include, but are not limited to, hydroxyl protecting groups known in the art, including glycols such as polyethylene glycol, tetraethylene glycol and the like.

The inhibitory action of the antisense nucleic acid can be examined using the transformant of the present invention, the gene expression system of the present invention in vivo and in vitro, or the translation system of the protein in vivo and in vitro. The nucleic acid can be applied to cells by a variety of publicly known methods.

Hereinafter, the protein of the present invention, its partial peptides, or salts thereof (hereinafter sometimes referred to as the protein of the present invention), the DNA encoding the protein of the present invention or its partial peptides (hereinafter sometimes referred to as the DNA of the present invention) and the antibodies to the protein of the present invention, its partial peptides, or salts thereof (hereinafter sometimes referred to as the antibodies of the present invention), etc. are specifically described for the applications.

The drug comprising a compound or its salt that inhibits the activity of the protein of the present invention can suppress the di- or tricarboxylic acid transport activity and can thus be used as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

On the other hand, the drug comprising a compound or its salt that promotes the activity of the protein of the present invention can promote the di- or tricarboxylic acid transport activity and can thus be used as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

### [1] Agents for the prevention/treatment of various diseases with which the protein of the invention is associated

The protein of the present invention has the di- or tricarboxylic acid transport activity, is responsible for the transport of succinate, citrate, malate, fumrate, a-ketoglutarate, etc. and at the same time, plays an important role in the metabolic response of cells.

Therefore, where the DNA encoding the protein of the present invention is abnormal or deficient, or where the expression level of the protein of the present invention is reduced, such causes a variety of diseases such as hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or causes the progress of aging.

Therefore, the protein of the present invention and the DNA of the present invention can be used as agents for the prevention/treatment of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, and as agents for the prevention/retardation of aging.

For example, when the transport of a di- or tricarboxylic acid cannot be expected in a patient sufficiently or normally due to a decrease or deficiency of the protein of the present invention in the body, the role of the protein of the present invention can be exhibited sufficiently or normally: (a) by administering the DNA of the present invention directly to the patient thereby to express the protein of the present invention; (b) by inserting the DNA of the present invention into cells to express the protein of the present invention and transplant the cells to the patient; or (c) by administering the protein of the present invention to the patient.

Where the DNA of the present invention is used as the agent for the prevention/treatment described above, the DNA of the present invention is administered singly; alternatively, the DNA is inserted into an appropriate vector such as retrovirus vector, adenovirus vector, adenovirus-associated virus vector, etc. and then administered to human or other warm-blooded animal in a conventional manner. The DNA of the present invention may also be administered as intact DNA, or with pharmacologically acceptable carrier such as adjuvants to assist its uptake by gene gun or through a catheter such as a catheter with a hydrogel.

When the protein of the present invention is used as the agent for the prevention/treatment described above, it is preferred to use the protein with a purity of at least 90%, preferably at least 95%, more preferably at least 98% and most preferably at least 99%.

The protein of the present invention can be used orally, for example, in the form of tablets which may be sugar coated if necessary, capsules, elixirs, microcapsules etc., or parenterally in the form of injectable preparations such as a sterile solution or suspension in water or other pharmaceutically acceptable liquid. These preparations can be manufactured by mixing the protein of the present invention with a physiologically acceptable carrier, a flavoring agent, an excipient, a vehicle, an antiseptic agent, a stabilizer, a binder, etc. in a unit dosage form required in a generally accepted manner that is applied to making pharmaceutical preparations. The active ingredient in these preparations is controlled in such a dose that an appropriate dose is obtained within the specified range given.

Additives miscible with tablets, capsules, etc. include a binder such as gelatin, corn starch, tragacanth and gum arabic, an excipient such as crystalline cellulose, a swelling agent such as corn starch, gelatin and alginic acid, a lubricant such as magnesium stearate, a sweetening agent such as sucrose, lactose and saccharin, a flavoring agent such as peppermint, akamono oil and cherry, and the like. When the unit dosage is in the form of capsules, liquid carriers such as oils and fats may further be used together with the additives described above. A sterile composition for injection may be formulated by conventional procedures used to make pharmaceutical preparations, e.g., by dissolving or suspending the active ingredients in a vehicle such as water for injection with a naturally occurring vegetable oil such as sesame oil and coconut oil, etc. to prepare the pharmaceutical preparations.

Examples of an aqueous medium for injection include physiological saline and an isotonic solution containing glucose and other auxiliary agents (e.g., D-sorbitol, D-mannitol, sodium chloride, etc.) and may be used in combination with an appropriate dissolution aid such as an alcohol (e.g., ethanol or the like), a polyalcohol (e.g., propylene glycol, polyethylene glycol, etc.), a nonionic surfactant (e.g., polysorbate 80™ , HCO-50, etc.), and the like. Examples of the oily medium include sesame oil and soybean oil, which may also be used in combination with a dissolution aid such as benzyl benzoate, benzyl alcohol, etc. The agent may further be formulated with a buffer (e.g., phosphate buffer, sodium acetate buffer, etc.), a soothing agent (e.g., benzalkonium chloride, procaine hydrochloride, etc.), a stabilizer (e.g., human serum albumin, polyethylene glycol, etc.), a preservative (e.g., benzyl alcohol, phenol, etc.), an antioxidant, etc. The thus-prepared liquid for injection is normally filled in an appropriate ampoule.

The vector inserted with the DNA of the present invention is prepared into pharmaceutical preparations as described above and normally provided for use parenterally.

Since the thus obtained pharmaceutical preparation is safe and low toxic, the preparation can be administered to warm-blooded animals (e.g., human, rats, mice, guinea pigs, rabbits, chicken, sheep, swine, bovine, horses, cats, dogs, monkeys, chimpanzees, etc.).

The dose of the protein of the present invention varies depending on target disease, subject to be administered, routes for administration, etc. When the protein of the present invention is orally administered to the patient, e.g., with hepatic disease, the protein, etc. is administered to adult (as 60 kg body weight) in a dose of normally about 0.1 mg to about 100 mg, preferably about 1.0 to about 50 mg, and more preferably about 1.0 to about 20 mg per day. In parenteral administration, the single dose of the protein, etc. varies depending on subject to be administered, target disease, etc. When the protein of the present invention is administered to adult (as 60 kg body weight) in the form of injection for the treatment of, e.g., hepatic disease, it is advantageous to administer to the patient by injecting the protein, etc. into the affected site in a daily dose of about 0.01 to about 30 mg, preferably about 0.1 to about 20 mg, and more preferably about 0.1 to about 10 mg. For other animal species, the corresponding dose as converted per 60 kg body weight can be administered.

### [2] Screening of drug candidate compounds for diseases

The protein of the present invention is useful as a reagent for screening a compound or its salt that promotes or inhibits the activity of the protein of the present invention.

The present invention provides (1) a method of screening a compound or its salt that promotes or inhibits the activity (e.g., di- or tricarboxylic acid transport, etc.) of the protein of the present invention (hereinafter sometimes merely referred to as the promoter or the inhibitor), which comprises using the protein of the present invention. In particular, the present invention provides, for example:

(2) a method of screening a promoter or inhibitor, which comprises comparing (i) the transport of a di- or tricarboxylic acid in cells capable of producing the protein of the present invention and (ii) the transport of a di- or tricarboxylic acid in a mixture of cells capable of producing the protein of the present invention and a test compound.

Specifically, the screening method described above is characterized by measuring the transport of a di- or tricarboxylic acid in the cases of (i) and (ii) using a labeled substrate (e.g., a fluorescence-labeled or radioisotope-labeled substrate) and comparing them in terms of the di- or tricarboxylic acid transport as an indicator.

For the test compound, for example, peptides, proteins, non-peptide compounds, synthetic compounds, fermentation products, cell extracts, plant extracts, animal tissue extracts, and the like are used. These compounds may be novel or known compounds.

To perform the screening method described above, cells capable of producing the protein of the present invention are suspended in a buffer suitable for the screening to prepare the cell suspension. Any buffer is usable as far as it is a buffer such as a phosphate buffer, borate buffer, etc. having pH of approximately 4 to 10 (preferably pH of about 6 to about 8) that does not interfere the di- or tricarboxylic acid transport of the protein of the present invention.

As the cell capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the cell membrane, e.g., by culturing through the procedure described above, is preferably employed.

The activities such as the di- or tricarboxylic acid transport, etc. may be assayed by publicly known methods with modifications, e.g., by the method described in Am. J. Physiol. Cell. Physiol., 278, C1019-C1030 (2000) or its modifications.

For example, when a test compound promotes the transport of a di- or tricarboxylic acid in the case (ii) described above by at least about 20%, preferably at least 30%, more preferably at least about 50%, as compared to the case (i) above, the test compound can be selected to be a compound capable of promoting the activity of the protein of the present invention, or a salt of said compound.

Also, for example, when a test compound inhibits (or suppresses) the transport of a di- or tricarboxylic acid in the case (ii) described above by at least about 20%, preferably at least 30%, more preferably at least about 50%, as compared to the case (i) above, the test compound can be selected to be a compound capable of inhibiting the activity of the protein of the present invention, or a salt of said compound.

Furthermore, the compound or its salt that promotes or suppresses the expression of the protein of the present invention (i.e., the compound or its salt that promotes or inhibits the activity of the protein of the present invention) can also be screened by inserting a gene such as secreted alkaline phosphatase, luciferase, etc. at the downstream of a promoter for a gene of the protein of the present invention thereby to express the gene in the various cells described above, and investigating such a compound or its salt that activates or inhibits the enzyme activity when a test compound as described above is brought in contact with the cells.

The polynucleotide encoding the protein of the present invention is useful as a reagent for screening the compound or its salt that promotes or inhibits a gene of the protein of the present invention.

The present invention provides (3) a method of screening a compound or its salt that promotes or inhibits the expression of a gene of the protein of the present invention (hereinafter sometimes merely referred to as a promoter or am inhibitor), which comprises using the polynucleotide encoding the protein of the present invention. In particular, the present invention provides, for example:

(4) a method of screening a promoter or an inhibitor, which comprises comparing (iii) the case where cells capable of producing the protein of the present invention are cultured and (iv) the case where a mixture of cells capable of producing the protein of the present invention and a test compound is cultured.

In the screening method described above, for example, the expression level of the gene of the protein of the present invention (specifically, the level of the protein of the present invention or the level of mRNA encoding said protein) is measured in the cases (iii) and (iv) and comparison is made therebetween.

For the test compound, for example, peptides, proteins, non-peptide compounds, synthetic compounds, fermentation products, cell extracts, plant extracts, animal tissue extracts, and the like are used. These compounds may be novel or known compounds.

To perform the screening method described above, cells capable of producing the protein of the present invention are suspended in a buffer suitable for the screening to prepare the cell suspension. Any buffer is usable as far as it is a buffer such as a phosphate buffer, borate buffer, etc. having pH of approximately 4 to 10 (preferably pH of about 6 to about 8), which does not interfere the organic anion transport activity of the protein of the present invention.

As the cell capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the cell membrane, e.g., by culturing through the procedure described above, is preferably employed.

The level of the protein of the present invention can be determined by publicly known methods, e.g., by measuring the aforesaid protein present in the cell extract, etc., using an antibody capable of recognizing the protein of the present invention, in accordance with methods like western blot analysis, ELISA, etc., or their modifications.

The expression level of the gene of the protein of the present invention can be determined by publicly known methods, e.g., in accordance with methods such as Northern blotting, reverse transcription-polymerase chain reaction(RT-PCR), real time PCR monitoring system (manufactured by ABI, TaqMan polymerase chain reaction), etc., or their modifications.

For example, when a test compound promotes the expression level of the gene of the protein of the present invention in the case (iv) described above by at least about 20%, preferably at least 30%, more preferably at least about 50%, as compared to the case (iii) above, the test compound can be selected to be a compound capable of promoting the expression of the protein gene of the present invention, or a salt of said compound.

For example, when a test compound inhibits the expression level of the gene of the protein of the present invention in the case (iv) described above by at least about 20%, preferably at least 30%, more preferably at least about 50%, as compared to the case (iii) above, the test compound can be selected to be a compound capable of inhibiting the expression of the protein gene of the present invention, or a salt of said compound.

In addition, the antibody of the present invention is useful as a reagent for . screening the compound or its salt that promotes or inhibits the expression of the protein of the present invention.

The present invention provides (5) a method of screening a compound or its salt that promotes or inhibits the expression of the protein of the present invention (hereinafter sometimes merely referred to as the promoter or the inhibitor), which comprises using the antibody of the present invention. In particular, the present invention provides, for example:

(6) a method of screening a promoter or inhibitor, which comprises comparing (i) the case where cells capable of producing the protein of the present invention are cultured and (ii) the case where a mixture of cells capable of producing the protein of the present invention and a test compound is cultured.

In the screening method described above, for example, the expression level of the protein of the present invention (specifically, the level of the protein of the present invention) is determined (e.g., detection of expression of the protein of the present invention is detected, quantification of the expression level of the protein of the present invention, etc.) in the cases (v) and (vi) using the antibody of the present invention, and comparison is made therebetween.

For the test compound, for example, peptides, proteins, non-peptide compounds, synthetic compounds, fermentation products, cell extracts, plant extracts, animal tissue extracts, and the like are used. These compounds may be novel or known compounds.

To perform the screening method described above, cells capable of producing the protein of the present invention are suspended in a buffer suitable for the screening to prepare the cell suspension. Any buffer is usable so long as it is a buffer such as a phosphate buffer, borate buffer, etc. having pH of approximately 4 to 10 (preferably pH of about 6 to about 8), which does not interfere the organic anion transport activity of the protein of the present invention.

As the cell capable of producing the protein of the present invention, there is employed, e.g., the aforesaid host (transformant) transformed with a vector containing the DNA encoding the protein of the present invention. Preferably, animal cells such as CHO cells, etc. are used as the host. For the screening, the transformant, in which the protein of the present invention has been expressed on the cell membrane, e.g., by culturing through the procedure described above, is preferably employed.

The level of the protein of the present invention can be determined by publicly known methods, e.g., by measuring the aforesaid protein present in the cell extract, etc., using an antibody capable of recognizing the protein of the present invention, in accordance with methods such as western blot analysis, ELISA, etc., or their modifications.

For example, when a test compound promotes the expression level of the protein of the present invention in the case (vi) described above by at least about 20%, preferably at least 30%, more preferably at least about 50%, as compared to the case (v) above, the test compound can be selected to be a compound capable of promoting the expression of the protein of the present invention, or a salt of said compound.

For example, when a test compound inhibits the expression level of the protein of the present invention in the case (vi) described above by at least about 20%, preferably at least 30%, more preferably at least about 50%, as compared to the case (v) above, the test compound can be selected to be a compound capable of inhibiting the expression of the protein of the present invention, or a salt of said compound.

The screening kit of the present invention comprises the protein of the present invention, its partial peptide, or a salt thereof, or the cell capable of producing the protein used in the present invention or its partial peptide.

The compound or its salt obtained using the screening method or screening kit of the present invention is the test compound described above, e.g., the compound selected from peptides, proteins, non-peptide compounds, synthetic compounds, fermentation products, cell extracts, plant extracts, animal tissue extracts, plasma, etc., which is a compound or its salt that promotes or inhibits the activity of the protein of the present invention (e.g., the di- or tricarboxylic acid transport, etc.).

The salts of these compounds used are those given above as the salts of the protein of the present invention.

The compound or its salt that promotes the activity of the protein of the present invention, the compound or its salt that promotes the expression of the protein gene of the present invention, or the compound or its salt that promotes the expression of the protein of the present invention is useful as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

Also, the compound or its salt that inhibits the activity of the protein of the present invention, the compound or its salt that inhibits the expression of the protein gene of the present invention, or the compound or its salt that inhibits the expression of the protein of the present invention is useful as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

When the compounds or their salt forms obtained by the screening methods or screening kits of the present invention are used as agents for the prevention/treatment or prevention/retardation described above, pharmaceutical preparations can be prepared following the conventional methods. For example, the compounds can be prepared into tablets, capsules, clixir, microcapsules, aseptic solution, suspension, etc.

Since the thus obtained pharmaceutical preparation is safe and low toxic, the preparation can be administered orally or parenterally to human or warm-blooded animals (e.g., mice, rats, rabbits, sheep, swine, bovine, horses, chicken, cats, dogs, monkeys, chimpanzees, etc.).

The dose of the compound or its salt varies depending on its action, target disease, subject to be administered, routes for administration, etc. When the compound or its salt that promotes the activity of the protein of the present invention is orally administered to adult (as 60 kg body weight) for the treatment of, e.g., hepatic disease, the compound of its salt is administered to the patient in a dose of normally about 0.1 mg to about 100 mg, preferably about 1.0 to about 50 mg, and more preferably about 1.0 to about 20 mg per day. In parenteral administration, the single dose of the compound or its salt varies depending on subject to be administered, target disease, etc. When the compound or its salt that promotes the activity of the protein of the present invention is administered to adult (as 60 kg body weight) in the form of injection for the treatment of, e.g., hepatic disease, the compound of its salt is intravenously administered to the patient in a daily dose of about 0.01 to about 30 mg, preferably about 0.1 to about 20 mg, and more preferably about 0.1 to about 10 mg. For other animal species, the corresponding dose as converted per 60 kg body weight can be administered.

### [3] Quantification of the protein of the present invention, its partial peptide, or its salt form

The antibodies to the protein of the present invention are capable of specifically recognizing the protein of the present invention. Therefore, the antibodies can be used to quantify the protein of the present invention in a test fluid, especially for quantification by the sandwich immunoassay.

That is, the present invention provides, for example, the following quantification methods:
(i) a method of quantifying the protein of the present invention in a test fluid, which comprises competitively reacting the antibody of the present invention with the test fluid and a labeled form of the protein of the present invention, and measuring the ratio of the labeled protein etc. bound to the antibody; and,
(ii) a method of quantifying the protein of the present invention in a test fluid, which comprises reacting the test fluid with the antibody of the present invention immobilized on a carrier and a labeled form of another antibody of the present invention simultaneously or sequentially, and measuring the activity of the label on the immobilized carrier.

In the quantification method (ii) described above, it is preferred that one antibody recognizes the N-terminal region of the protein of the present invention, and another antibody reacts with the C-terminal region of the protein of the present invention.

Using monoclonal antibodies to the protein of the present invention (hereinafter sometimes referred to as the monoclonal antibodies of the present invention), the protein of the present invention can be quantified and can also be detected by tissue staining or the like. For these purposes, an antibody molecule itself may be used, or F(ab')₂, Fab' or Fab fractions of the antibody molecule may also be used.

Methods for quantifying the protein of the present invention using the antibodies of the present invention are not particularly limited. Any method can be used, so long as the amount of antibody, antigen, or antibody-antigen complex corresponding to the amount of antigen (e.g., the amount of the protein) in a test fluid can be detected by chemical or physical means and the amount of the antigen can be calculated from a standard curve prepared from standard solutions containing known amounts of the antigen. For example, nephrometry, competitive methods, immunometric method, and sandwich method are appropriately used, with the sandwich method described below being most preferable in terms of sensitivity and specificity.

As the labeling agent for the methods using labeled substances, there are employed, for example, radioisotopes, enzymes, fluorescent substances, luminescent substances, etc. For the radioisotope, for example, [¹²⁵I], [¹³¹I], [³H], [¹⁴C], etc. are used. As the enzyme described above, stable enzymes with high specific activity are preferred; for example, β-galactosidase, β-glucosidase, alkaline phosphatase, peroxidase, malate dehydrogenase and the like are used. Examples of the fluorescent substance used are fluorescamine, fluorescein isothiocyanate, etc. are used. For the luminescent substance, there are employed, for example, luminol, luminol derivatives, luciferin, lucigenin, etc. Furthermore, the biotin-avidin system may be used for binding antibody or antigen to the label.

For immobilization of antigens or antibodies, physical adsorption may be used. Chemical binding methods conventionally used for insolubilization or immobilization of proteins, enzymes, etc. may also be used. For the carrier, there are, e.g., insoluble polysaccharides such as agarose, dextran, cellulose, etc.; synthetic resin such as polystyrene, polyacrylamide, silicon, etc., glass, and the like.

In the sandwich method, an immobilized form of the monoclonal antibody of the present invention is reacted with a test fluid (primary reaction), then with a labeled form of another monoclonal antibody of the present invention (secondary reaction), and the activity of the label on the immobilizing carrier is measured, whereby the amount of the protein of the present invention in the test fluid can be quantified. The order of the primary and secondary reactions may be reversed, and the reactions may be performed simultaneously or with an interval. The methods of labeling and immobilization can be performed by the methods described above. In the immunoassay by the sandwich method, the antibody used for immobilized or labeled antibodies is not necessarily one species, but a mixture of two or more species of antibody may be used to increase the measurement sensitivity.

In the methods of assaying the protein of the present invention by the sandwich method, antibodies that bind to different sites of the protein etc. are preferably used as the monoclonal antibodies of the present invention for the primary and secondary reactions. That is, in the antibodies used for the primary and secondary reactions are, for example, when the antibody used in the secondary reaction recognizes the C-terminal region of the protein, it is preferable to use the antibody recognizing the region other than the C-terminal region for the primary reaction, e.g., the antibody recognizing the N-terminal region.

The monoclonal antibodies of the present invention can be used for the assay systems other than the sandwich method, for example, competitive method, immunometric method, nephrometry, etc. In the competitive method, antigen in a test fluid and the labeled antigen are competitively reacted with antibody, and the unreacted labeled antigen (F) and the labeled antigen bound to the antibody (B) are separated (B/F separation). The amount of the label in B or F is measured, and the amount of the antigen in the test fluid is quantified. This reaction method includes a liquid phase method using a soluble antibody as an antibody, polyethylene glycol for B/F separation and a secondary antibody to the soluble antibody, and an immobilized method either using an immobilized antibody as the primary antibody, or using a soluble antibody as the primary antibody and immobilized antibody as the secondary antibody.

In the immunometric method, antigen in a test fluid and immobilized antigen are competitively reacted with a definite amount of labeled antibody, the immobilized phase is separated from the liquid phase, or antigen in a test fluid and an excess amount of labeled antibody are reacted, immobilized antigen is then added to bind the unreacted labeled antibody to the immobilized phase, and the immobilized phase is separated from the liquid phase. Then, the amount of the label in either phase is measured to quantify the antigen in the test fluid.

In the nephrometry, insoluble precipitate produced after the antigen-antibody reaction in gel or solution is quantified. When the amount of antigen in the test fluid is small and only a small amount of precipitate is obtained, laser nephrometry using scattering of laser is advantageously employed.

For applying these immunological methods to the measurement methods of the present invention, any particular conditions or procedures are not required. Systems for measuring the protein of the present invention are constructed by adding the usual technical consideration in the art to the conventional conditions and procedures. For the details of these general technical means, reference can be made to the following reviews and texts.

Reference can be made on, e.g., Hiroshi Irie, ed. "Radioimmunoassay" (Kodansha, published in 1974), Hiroshi Irie, ed. "Sequel to the Radioimmunoassay" (Kodansha, published in 1979), Eiji Ishikawa, et al. ed. "Enzyme immunoassay" (Igakushoin, published in 1978), Eiji Ishikawa, et al. ed. "Immunoenzyme assay" (2nd ed.) (Igakushoin, published in 1982), Eiji Ishikawa, et al. ed. "Immunoenzyme assay" (3rd ed.) (Igakushoin, published in 1987), Methods in ENZYMOLOGY, Vol. 70 (Immunochemical Techniques (Part A)), ibid., Vol. 73 (Immunochemical Techniques (Part B)), ibid., Vol. 74 (Immunochemical Techniques (Part C)), ibid., Vol. 84 (Immunochemical Techniques (Part D: Selected Immunoassays)), ibid., Vol. 92 (Immunochemical Techniques (Part E: Monoclonal Antibodies and General Immunoassay Methods)), ibid., Vol. 121 (Immunochemical Techniques (Part I: Hybridoma Technology and Monoclonal Antibodies)) (all published by Academic Press Publishing), etc.

As described above, the protein of the present invention can be quantified with high sensitivity, using the antibodies of the present invention.

When a decreased level of the protein of the present invention is detected by quantifying the level of the protein of the present invention using the antibody of the present invention, it can be diagnosed to be highly suspected of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome; renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or rapid progress of aging. Also, when an increased level of the protein of the present invention is detected, it can be diagnosed to be highly suspected of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or rapid progress of aging.

The antibodies of the present invention can also be used for specifically detecting the protein of the present invention present in test samples such as body fluids or tissues. The protein of the present invention may also be used for preparation of antibody columns used to purify the protein of the present invention, for detection of the protein of the present invention in each fraction upon purification, and for analysis of the behavior of the protein of the present invention in cells tested.

### [4] Gene diagnostic agent

By using the DNA of the present invention as a probe, an abnormality (gene abnormality) of the DNA or mRNA encoding the protein of the present invention or its partial peptide in human or other warm-blooded animals (e.g., rats, mice, guinea pigs, rabbits, chicken, sheep, swine, bovine, horses, cats, dogs, monkeys, chimpanzees, etc.) can be detected. Therefore, the DNA of the present invention is useful as a gene diagnostic agent for damages against the DNA or mRNA, its mutation, or its decreased expression, or increased expression or overexpression of the DNA or mRNA; etc.

The gene diagnosis described above using the DNA of the present invention can be performed by, for example, publicly known Northern hybridization or the PCR-SSCP assay (Genomics, 5, 874-879 (1989); Proceedings of the National Academy of Sciences of the United States of America, 86, 2766-2770 (1989)), etc.

When increased expression is detected by, e.g., Northern hybridization, it can be diagnosed to be highly suspected of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or rapid progress of aging. Also, when decreased expression is detected or mutation of DNA is detected by the PCR-SSCP assay, it can be diagnosed to be highly suspected of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or rapid progress of aging.

### [5] A drug comprising the antisense polynucleotide

Since the antisense polynucleotide of the present invention, which can complementarily bind to the DNA of the present invention and can suppress expression of the said DNA, is low toxic and can suppress an in vivo function or activity of the protein of the present invention or the DNA of the present invention (e.g., the di- or tricarboxylic acid transport, etc.), it can be used as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

When the antisense polynucleotide described above is used as an agent for the prevention/treatment described above, pharmaceutical preparations can be prepared and administered by following the conventional methods.

For example, where the antisense polynucleotide described above is used, the antisense polynucleotide is administered singly; alternatively, the antisense polynucleotide is inserted into an appropriate vector such as retrovirus vector, adenovirus vector, adenovirus-associated virus vector, etc. and then administered orally or parenterally to human or other mammals (e.g., rats, rabbits, sheep, swine, bovine, cats, dogs, monkeys, etc.) in a conventional manner. The antisense polynucleotide may also be administered as it is, or with pharmacologically acceptable carrier such as adjuvants to assist its uptake by gene gun or through a catheter such as a catheter with a hydrogel.

The dose of the antisense polynucleotide varies depending on target disease, subject to be administered, routes for administration, etc. When the antisense polynucleotide is administered topically to the liver for the treatment of, e.g., hepatic disease, the antisense polynucleotide is administered to adult (as 60 kg body weight) normally in a daily dose of about 0.1 mg to about 100 mg.

In addition, the antisense polynucleotide may also be used as a diagnostic oligonucleotide probe to investigate the presence of the DNA of the present invention or the state of its expression in tissues or cells.

The present invention further provides:
(1) double-stranded RNA containing a part of RNA encoding the protein of the present invention and a complementary RNA to the same;
(2) a drug comprising the aforementioned double-stranded RNA;
(3) a ribozyme containing a part of RNA encoding the protein of the present invention;
(4) a drug comprising the said ribozyme; and,
(5) an expression vector containing a gene (DNA) encoding the said ribozyme.

Since the double-stranded RNA, ribozyme, etc. can destroy RNA transcribed from the DNA of the present invention or can suppress its function as well as the antisense polynucleotide described above, they can suppress the in vivo function of the protein used in the present invention or the DNA used in the present invention; they can be used as agents for the prevention/treatment of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like.

The double-stranded RNA can be designed based on a sequence of the polynucleotide of the present invention and manufactured according to a publicly known method (e.g., Nature, 411, 494, 2001) with modifications thereof.

The ribozyme can be designed based on a sequence of the polynucleotide of the present invention and manufactured according to a publicly known method (e.g., TRENDS in Molecular Medicine, 7, 221, 2001) with modifications thereof. For example, it can be manufactured by replacing a part of RNA encoding the protein of the present invention for a part of ribozyme publicly known. The part of RNA encoding the protein of the present invention includes a sequence adjacent to consensus sequence NUX (wherein N represents all bases and X represents bases other than G), which may be cleaved by a publicly known ribozyme, etc.

Where the double-stranded RNA or ribozyme described above is used as the agent for the prevention/treatment described above, pharmaceutical preparations can be prepared and administered as in the case of the antisense polynucleotide. The expression vector (5) described above is used as in publicly known genetic therapy and provided for use as an agent for the prevention/treatment above.

### [6] Preparation of transgenic animal bearing the DNA of the present invention

The present invention provides a non-human mammal bearing an exogenous DNA encoding the protein of the present invention (hereinafter merely referred to as the exogenous DNA of the present invention) or its variant DNA (sometimes simply referred to as the exogenous variant DNA of the present invention).

Thus, the present invention provides:
(1) a non-human mammal bearing the exogenous DNA of the present invention or its variant DNA;
(2) the mammal according to (1), wherein the non-human mammal is a rodent;
(3) the mammal according to (2), wherein the rodent is mouse or rat; and,
(4) a recombinant vector bearing the exogenous DNA of the present invention or its variant DNA and capable of expressing in a mammal.

The non-human mammal bearing the exogenous DNA of the present invention or its variant DNA (hereinafter simply referred to as the DNA transgenic animal of the present invention) can be prepared by transfecting a desired DNA into an unfertilized egg, a fertilized egg, a spermatozoon, a germinal cell containing a primordial germinal cell thereof, or the like, preferably in the embryogenic stage in the development of a non-human mammal (more preferably in the single cell or fertilized cell stage and generally before the 8-cell phase), by standard means, such as the calcium phosphate method, the electric pulse method, the lipofection method, the agglutination method, the microinjection method, the particle gun method, the DEAE-dextran method etc. Also, it is possible to transfect the exogenous DNA of the present invention into a somatic cell, a living organ, a tissue cell, or the like by the DNA transfection methods and utilize the transformant for cell culture, tissue culture, etc. In addition, these cells may be fused with the above-described germinal cell by a publicly known cell fusion method to create the DNA transgenic animal of the present invention.

Examples of the non-human mammal that can be used include bovine, swine, sheep, goats, rabbits, dogs, cats, guinea pigs, hamsters, mice, rats, and the like. Above all, preferred are rodents, especially mice (e.g., C57BL/6 strain, DBA2 strain, etc. for a pure line and for a cross line, B6C3F₁ strain, BDF₁ strain B6D2F₁ strain, BALB/c strain, ICR strain, etc.) or rats (Wistar, SD, etc.), since they are relatively short in ontogeny and life cycle from a standpoint of creating model animals for human disease.

"Mammals" in a recombinant vector that can be expressed in the mammals include the aforesaid non-human mammals and human.

The exogenous DNA of the present invention refers to the DNA of the present invention that is once isolated/extracted from mammals, not the DNA of the present invention inherently possessed by the non-human mammals.

The variant DNA of the present invention includes variants resulting from variation (e.g., mutation, etc.) in the base sequence of the original DNA of the present invention, specifically DNAs resulting from base addition, deletion, substitution with other bases, etc. and further including abnormal DNA.

The abnormal DNA is intended to mean such a DNA that expresses the protein of the present invention, which is abnormal, and exemplified by the DNA that expresses a protein to suppress the functions of the normal protein of the present invention, or the like.

The exogenous DNA of the present invention may be any one of those derived from a mammal of the same species as, or a different species from, the mammal as the target animal. In transfecting the DNA of the present invention, it is generally advantageous to use the DNA as a DNA construct in which the DNA is ligated downstream a promoter capable of expressing the DNA in the target animal. For example, in the case of transfecting the human DNA of the present invention, a DNA transgenic mammal that expresses the DNA of the present invention to a high level, can be prepared by microinjecting a DNA construct (e.g., vector, etc.) ligated with the human DNA of the present invention into a fertilized egg of the target non-human mammal downstream various promoters, which are capable of expressing the DNA derived from various mammals (e.g., rabbits, dogs, cats, guinea pigs, hamsters, rats, mice, etc.) bearing the DNA of the present invention highly homologous to the human DNA.

As expression vectors for the protein of the present invention, there are *Escherichia coli*-derived plasmids, *Bacillus subtilis*-derived plasmids, yeast-derived plasmids, bacteriophages such as λ phage, retroviruses such as Moloney leukemia virus, etc., and animal viruses such as vaccinia virus, baculovirus, etc. Of these vectors, *Escherichia coli*-derived plasmids, *Bacillus subtilis*-derived plasmids, or yeast-derived plasmids, etc. are preferably used.

Examples of these promoters for regulating the DNA expression described above include (i) promoters for the DNA derived from viruses (e.g., simian virus, cytomegalovirus, Moloney leukemia virus, JC virus, breast cancer virus, poliovirus, etc.), and (ii) promoters derived from various mammals (human, rabbits, dogs, cats, guinea pigs, hamsters, rats, mice, etc.), for example, promoters of albumin, insulin II, uroplakin II, elastase, erythropoietin, endothelin, muscular creatine kinase, glial fibrillary acidic protein, glutathione S-transferase, platelet-derived growth factor β, keratins K1, K10 and K14, collagen types I and II, cyclic AMP-dependent protein kinase βI subunit, dystrophin, tartarate-resistant alkaline phosphatase, atrial natriuretic factor, endothelial receptor tyrosine kinase (generally abbreviated as Tie2), sodium-potassium adenosine triphosphorylase (Na,K-ATPase), neurofilament light chain, metallothioneins I and IIA, metalloproteinase I tissue inhibitor, MHC class I antigen (H-2L), H-ras, renin, dopamine β-hydroxylase, thyroid peroxidase (TPO), polypeptide chain elongation factor 1α (EF-1α), β actin, α and β myosin heavy chains, myosin light chains 1 and 2, myelin base protein, thyroglobulins, Thy-1, immunoglobulins, H-chain variable region (VNP), serum amyloid component P, myoglobin, troponin C, smooth muscle α actin, preproencephalin A, vasopressin, etc. Among others them, cytomegalovirus promoters, human polypeptide elongation factor 1α (EF-1α) promoters, human and chicken β actin promoters etc., which protein can highly express in the whole body are preferred.

It is preferred that the vectors described above have a sequence for terminating the transcription of the desired messenger RNA in the DNA transgenic animal (generally called a terminator); for example, a sequence of each DNA derived from viruses and various mammals. SV40 terminator of simian virus, etc. is preferably used.

In addition, for the purpose of increasing the expression of the desired exogenous DNA to a higher level, the splicing signal and enhancer region of each DNA, a portion of the intron of an eukaryotic DNA may also be ligated at the 5' upstream of the promoter region, or between the promoter region and the translational region, or at the 3' downstream of the translational region, depending upon purpose.

The normal translational region for the protein of the present invention can be obtained using as a starting material the entire genomic DNA or its portion of liver, kidney, thyroid cell or fibroblast origin from human or various mammals (e.g., rabbits, dogs, cats, guinea pigs, hamsters, rats, mice, etc.) or of various commercially available genomic DNA libraries, or using complementary DNA prepared by a publicly known method from RNA of liver, kidney, thyroid cell or fibroblast origin as a starting material. Also, an exogenous abnormal DNA can produce a translational region, which is obtained by point mutagenesis variation of the normal translational region for a polypeptide obtained from the cells or tissues described above.

The said translational region can be prepared by a conventional DNA engineering technique, in which the DNA is ligated downstream the aforesaid promoter and if desired, upstream the translation termination site, as a DNA construct capable of being expressed in the transgenic animal.

The exogenous DNA of the present invention is transfected at the fertilized egg cell stage in a manner such that the DNA is certainly present in all the germinal cells and somatic cells of the target mammal. The fact that the exogenous DNA of the present invention is present in the germinal cells of the animal prepared by DNA transfection means that all offspring of the prepared animal will maintain the exogenous DNA of the present invention in all of the germinal cells and somatic cells thereof. The offspring of the animal that inherits the exogenous DNA of the present invention also have the exogenous DNA of the present invention in all of the germinal cells and somatic cells thereof.

The non-human mammal, in which the normal exogenous DNA of the present invention has been transfected, can be passaged as the DNA-bearing animal under ordinary rearing environment, by confirming that the exogenous DNA is stably retained by mating.

By the transfection of the exogenous DNA of the present invention at the fertilized egg cell stage, the DNA is retained to be excess in all of the germinal and somatic cells. The fact that the exogenous DNA of the present invention is excessively present in the germinal cells of the prepared animal after transfection means that the exogenous DNA of the present invention is excessively present in all of the germinal cells and somatic cells thereof. The offspring of the animal that inherits the exogenous DNA of the present invention have excessively the exogenous DNA of the present invention in all of the germinal cells and somatic cells thereof.

By acquiring a homozygotic animal having the transfected DNA in both of homologous chromosomes and mating a male and female of the animal, all offspring can be passaged to retain the DNA.

In a non-human mammal bearing the normal DNA of the present invention, the normal DNA of the present invention has expressed to a high level, and may eventually develop the hyperfunction of the protein of the present invention by promoting the function of endogenous normal DNA. Therefore, the animal can be utilized as a pathologic model animal for such a disease. Specifically, using the normal DNA transgenic animal of the present invention, it is possible to elucidate the mechanism of the hyperfunction of the protein of the present invention and the pathological mechanism of the disease associated with the protein of the present invention and to determine how to treat the disease.

Furthermore, since a mammal wherein the exogenous normal DNA of the present invention is transfected exhibits an increasing symptom of the protein of the present invention librated, the animal is usable for screening therapeutic agents for the disease associated with the protein of the present invention.

On the other hand, a non-human mammal having the exogenous abnormal DNA of the present invention can be passaged under normal breeding conditions as the DNA-bearing animal by confirming the stable retaining of the exogenous DNA via crossing. Further, the exogenous DNA to be subjected can be utilized as a starting material by inserting the DNA into the plasmid described above. The DNA construct with promoter can be prepared by conventional DNA engineering techniques. The transfection of the abnormal DNA of the present invention at the fertilized egg cell stage is preserved to be present in all of the germinal and somatic cells of the mammals to be subjected. The fact that the abnormal DNA of the present invention is present in the germinal cells of the animal after DNA transfection means that all of the offspring of the prepared animal have the abnormal DNA of the present invention in all of the germinal and somatic cells. Such an offspring passaged the exogenous DNA of the present invention contains the abnormal DNA of the present invention in all of the germinal and somatic cells. A homozygous animal having the introduced DNA on both of homologous chromosomes can be acquired and then by mating these male and female animals, all the offspring can be bled to have the DNA.

Since a non-human mammal having the abnormal DNA of the present invention may express the abnormal DNA of the present invention at a high level, the animal may be the function inactivation type inadaptability of the protein of the present invention by inhibiting the function of the endogenous normal DNA and can be utilized as its disease model animal. For example, using the abnormal DNA-transgenic animal of the present invention, it is possible to elucidate the mechanism of inadaptability of the protein of the present invention and to perform to study a method for treatment of this disease.

More specifically, the transgenic animal of the present invention expressing the abnormal DNA of the present invention to a high level is also expected to serve as an experimental model for the elucidation of the mechanism of the functional inhibition (dominant negative effect) of normal protein by the abnormal protein of the present invention in the function inactive type inadaptability of the protein of the present invention.

A mammal bearing the abnormal exogenous DNA of the present invention is also expected to serve for screening a candidate drug for the treatment of the function inactive type inadaptability of the protein of the present invention, since the protein of the present invention is increased in such an animal in its free form.

Other potential applications of two kinds of the transgenic animals described above include:
(i) use as a cell source for tissue culture;
(ii) elucidation of the relation to a protein that is specifically expressed or activated by the protein of the present invention, by direct analysis of DNA or RNA in tissue of the DNA transgenic animal of the present invention or by analysis of the polypeptide tissue expressed by the DNA;
(iii) research in the function of cells derived from tissues that are cultured usually only with difficulty, using cells of tissue bearing the DNA cultured by a standard tissue culture technique;
(iv) screening of a drug that enhances the functions of cells using the cells described in (iii) above; and,
(v) isolation and purification of the variant protein of the present invention and preparation of an antibody thereto.

Furthermore, clinical conditions of a disease associated wit the protein of the present invention, including the function inactive type inadaptability of the protein of the present invention can be determined using the DNA transgenic animal of the present invention. Also, pathological findings on each organ in a disease model associated with the protein of the present invention can be obtained in more detail, leading to the development of a new method for treatment as well as the research and therapy of any secondary diseases associated with the disease.

It is also possible to obtain a free DNA-transfected cell by withdrawing each organ from the DNA transgenic animal of the present invention, mincing the organ and degrading with a proteinase such as trypsin, etc., followed by establishing the line of culturing or cultured cells. Furthermore, the DNA transgenic animal of the present invention can serve as identification of cells capable of producing the protein of the present invention, and as studies on association with apoptosis, differentiation or propagation or on the mechanism of signal transduction in these properties to inspect any abnormality therein. Thus, the DNA transgenic animal of the present invention can provide an effective research material for the protein of the present invention and for elucidating the function and effects thereof.

To develop a therapeutic drug for the treatment of diseases associated with the protein of the present invention, including the function inactive type inadaptability of the protein of the present invention, using the DNA transgenic animal of the present invention, an effective and rapid method for screening can be provided by using the method for inspection and the method for quantification, etc. described above. It is also possible to investigate and develop a method for DNA therapy for the treatment of diseases associated with the protein of the present invention, using the DNA transgenic animal of the present invention or a vector capable of expressing the exogenous DNA of the present invention.

### [7] Knockout animal

The present invention provides a non-human mammal embryonic stem cell bearing the DNA of the present invention inactivated and a non-human mammal deficient in expressing the DNA of the present invention.

Thus, the present invention provides:
(1) a non-human embryonic stem cell in which the DNA of the present invention is inactivated;
(2) an embryonic stem cell according to (1), wherein the DNA is inactivated by introducing a reporter gene (e.g., β-galactosidase gene derived from *Escherichia coli*);
(3) an embryonic stem cell according to (1), which is resistant to neomycin;
(4) an embryonic stem cell according to (1), wherein the non-human mammal is a rodent;
(5) an embryonic stem cell according to (4), wherein the rodent is mouse;
(6) a non-human mammal deficient in expressing the DNA of the present invention, wherein the DNA of the present invention is inactivated;
(7) a non-human mammal according to (6), wherein the DNA is inactivated by inserting a reporter gene (e.g., β-galactosidase derived from *Escherichia coli*) therein and the reporter gene is capable of being expressed under control of a promoter for the DNA of the present invention;
(8) a non-human mammal according to (6), which is a rodent;
(9) a non-human mammal according to (8), wherein the rodent is mouse; and,
(10) a method of screening a compound or its salt that promotes or inhibits the promoter activity for the DNA of the present invention, which comprises administering a test compound to the mammal of (7) and detecting expression of the reporter gene.

The non-human mammal embryonic stem cell in which the DNA of the present invention is inactivated refers to a non-human mammal embryonic stem cell that suppresses the ability of the non-human mammal to express the DNA by artificially mutating the DNA of the present invention, or the DNA has no substantial ability to express the protein of the present invention (hereinafter sometimes referred to as the knockout DNA of the present invention) by substantially inactivating the activity of the protein of the present invention encoded by the DNA (hereinafter merely referred to as ES cell).

As the non-human mammal, there are employed the same examples as given above.

Techniques for artificially mutating the DNA of the present invention include deletion of a part or all of the DNA sequence and insertion of or substitution with other DNA, by genetic engineering. By these variations, the knockout DNA of the present invention may be prepared, for example, by shifting the reading frame of a codon or by disrupting the function of a promoter or exon.

Specifically, the non-human mammal embryonic stem cell in which the DNA of the present invention is inactivated (hereinafter merely referred to as the ES cell with the DNA of the present invention inactivated or the knockout ES cell of the present invention) can be obtained by, for example, isolating the DNA of the present invention that the desired non-human mammal possesses, inserting a DNA fragment having a DNA sequence constructed by inserting a drug resistant gene such as a neomycin resistant gene or a hygromycin resistant gene, or a reporter gene such as lacZ (β-galactosidase gene) or cat (chloramphenicol acetyltransferase gene), etc. into its exon site thereby to disable the functions of exon, or integrating to a chromosome of the subject animal by, e.g., homologous recombination, a DNA sequence which terminates gene transcription (e.g., polyA additional signal, etc.) in the intron between exons, thus inhibiting the synthesis of complete messenger RNA to eventually destroy the gene (hereinafter simply referred to as targeting vector). The thus obtained ES cells are subjected to Southern hybridization analysis using a DNA sequence on or near the DNA of the present invention as a probe, or to PCR analysis using a DNA sequence on the targeting vector and another DNA sequence near the DNA of the present invention, which is not included in the targeting vector as primers, thereby to select the knockout ES cell of the present invention.

The parent ES cells to inactivate the DNA of the present invention by homologous recombination, etc. may be of a strain already established as described above, or may be originally established in accordance with a modification of the known method by Evans and Kaufman *supra.* For example, in the case of mouse ES cells, currently it is common practice to use ES cells of the 129 strain. However, since their immunological background is obscure, the C57BL/6 mouse or the BDF₁ mouse (F₁ hybrid between C57BL/6 and DBA/2), wherein the low ovum availability per C57BL/6 in the C57BL/6 mouse has been improved by crossing with DBA/2, may be preferably used, instead of obtaining a pure line of ES cells with the clear immunological genetic background and for other purposes. The BDF₁ mouse is advantageous in that, when a pathologic model mouse is generated using ES cells obtained therefrom, the genetic background can be changed to that of the C57BL/6 mouse by back-crossing with the C57BL/6 mouse, since its background is of the C57BL/6 mouse, as well as being advantageous in that ovum availability per animal is high and ova are robust.

In establishing ES cells, blastocytes at 3.5 days after fertilization are commonly used. In the present invention, embryos are preferably collected at the 8-cell stage, after culturing until the blastocyte stage, the embryos are used to efficiently obtain a large number of early stage embryos.

Although the ES cells used may be of either sex, male ES cells are generally more convenient for generation of a germ cell line chimera and are therefore preferred. It is also desirable that sexes are identified as soon as possible to save painstaking culture time.

Methods for sex identification of the ES cell include the method in which a gene in the sex-determining region on the Y-chromosome is amplified by the PCR process and detected. When this method is used, one colony of ES cells (about 50 cells) is sufficient for sex-determination analysis, which karyotype analysis, for example G-banding method, requires about 10⁶ cells; therefore, the first selection of ES cells at the early stage of culture can be based on sex identification, and male cells can be selected early, which saves a significant amount of time at the early stage of culture.

Second selection can be achieved by, for example, number of chromosome confirmation by the G-banding method. It is usually desirable that the chromosome number of the obtained ES cells be 100% of the normal number. However, when it is difficult to obtain the cells having the normal number of chromosomes due to physical operation etc. in cell establishment, it is desirable that the ES cell be again cloned to a normal cell (e.g., in mouse cells having the number of chromosomes being 2n = 40) after the gene of the ES cells is rendered knockout.

Although the embryonic stem cell line thus obtained shows a very high growth potential, it must be subcultured with great care, since it tends to lose its ontogenic capability. For example, the embryonic stem cell line is cultured at about 37°C in a carbon dioxide incubator (preferably about 5% carbon dioxide and about 95% air, or about 5% oxygen, about 5% carbon dioxide and 90% air) in the presence of LIF (1-10000 U/ml) on appropriate feeder cells such as STO fibroblasts, treated with a trypsin/EDTA solution (normally about 0.001 to about 0.5% trypsin/about 0.1 to about 5 mM EDTA, preferably about 0.1% trypsin/1 mM EDTA) at the time of passage to obtain separate single cells, which are then seeded on freshly prepared feeder cells. This passage is normally conducted every 1 to 3 days; it is desirable that cells are observed at passage and cells found to be morphologically abnormal in culture, if any, should be abandoned.

Where ES cells are allowed to reach a high density in mono-layers or to form cell aggregates in suspension under appropriate conditions, they will spontaneously differentiate to various cell types, for example, pariental and visceral muscles, cardiac muscle or the like [M. J. Evans and M. H. Kaufman, Nature, 292, 154, 1981; G. R. Martin, Proc. Natl. Acad. Sci. U.S.A., 78, 7634, 1981; T. C.

Doetschman et al., Journal of Embryology Experimental Morphology, 87, 27, 1985]. The cells deficient in expressing the DNA of the present invention, which are obtained from the differentiated ES cells of the present invention, are useful for studying the protein of the present invention in vitro from an aspect of cell biology.

The non-human mammal deficient in expressing the DNA of the present invention can be distinguished from a normal animal by measuring the mRNA amount in the subject animal by a publicly known method, and indirectly comparing the levels of expression.

As the non-human mammal, there are employed the same examples as described above.

With respect to the non-human mammal deficient in expressing the DNA of the present invention, the DNA of the present invention can be rendered knockout by transfecting a targeting vector, prepared as described above, to non-human mammal embryonic stem cells or oocytes thereof, and conducting homologous recombination in which a targeting vector DNA sequence, wherein the DNA of the present invention is inactivated by the transfection, is replaced with the DNA of the present invention on a chromosome of a non-human mammal embryonic stem cell or embryo thereof.

The cells with the DNA of the present invention knockout can be identified by Southern hybridization analysis using a DNA sequence on or near the DNA of the present invention as a probe, or by PCR analysis using as primers a DNA sequence on the targeting vector and another DNA sequence, which is not included in the targeting vector. When non-human mammalian embryonic stem cells are used, a cell line wherein the DNA of the present invention is inactivated by homologous recombination is cloned; the resulting cloned cell line is injected to, e.g., a non-human mammalian embryo or blastocyte, at an appropriate stage such as the 8-cell stage. The resulting chimeric embryos are transplanted to the uterus of the pseudopregnant non-human mammal. The resulting animal is a chimeric animal composed of both cells having the normal locus of the DNA of the present invention and those having an artificially mutated locus of the DNA of the present invention.

When some germ cells of the chimeric animal have a mutated locus of the DNA of the present invention, an individual, which entire tissue is composed of cells having a mutated locus of the DNA of the present invention can be selected from a series of offspring obtained by crossing between such a chimeric animal and a normal animal, e.g., by coat color identification, etc. The individuals thus obtained are normally deficient in heterozygous expression of the protein of the present invention. The individuals deficient in homozygous expression of the protein of the present invention can be obtained from offspring of the intercross between the heterozygotes of the protein of the present invention.

When an oocyte or egg cell is used, a DNA solution may be injected, e.g., to the prenucleus by microinjection thereby to obtain a transgenic non-human mammal having a targeting vector introduced in a chromosome thereof. From such transgenic non-human mammals, those having a mutation at the locus of the DNA of the present invention can be obtained by selection based on homologous recombination.

As described above, individuals in which the DNA of the present invention is rendered knockout permit passage rearing under ordinary rearing conditions, after the individuals obtained by their crossing have proven to have been knockout.

Furthermore, the genital system may be obtained and maintained by conventional methods. That is, by crossing male and female animals each having the inactivated DNA, homozygote animals having the inactivated DNA in both loci can be obtained. The homozygotes thus obtained may be reared so that one normal animal and two or more homozygotes are produced from a mother animal to efficiently obtain such homozygotes. By crossing male and female heterozygotes, homozygotes and heterozygotes having the inactivated DNA are proliferated and passaged.

The non-human mammal embryonic stem cell, in which the DNA of the present invention is inactivated, is very useful for preparing a non-human mammal deficient in expressing the DNA of the present invention.

Since the non-human mammal deficient in expressing the DNA of the present invention lacks various biological activities derived from the protein of the present invention, such an animal can be a disease model suspected of inactivated biological activities of the protein of the present invention and thus, offers an effective study to investigate causes for and treatment for these diseases.

### [7a] Method for screening of compounds having therapeutic/preventive effects on diseases caused by deficiency, damages, etc. of the DNA of the present invention

The non-human mammal deficient in expressing the DNA of the present invention can be employed for screening of compounds having therapeutic/prophylactic effects on diseases caused by deficiency, damages, etc. of the DNA of the present invention.

That is, the present invention provides a method for screening of a compound or its salt having therapeutic/preventive effects on diseases caused by deficiency, damages, etc. of the DNA of the present invention, which comprises administering a test compound to the non-human mammal deficient in expressing the DNA of the present invention and observing/measuring a change occurred in the animal.

As the non-human mammal deficient in expressing the DNA of the present invention which can be employed for the screening method, the same examples as given hereinabove apply.

Examples of the test compounds include peptides, proteins, non-peptide compounds, synthetic compounds, fermentation products, cell extracts, vegetable extracts, animal tissue extracts, blood plasma, etc. These compounds may be novel compounds or publicly known compounds.

Specifically, the non-human mammal deficient in expressing the DNA of the present invention is treated with a test compound, comparison is made with an intact animal for control and a change in each organ, tissue, disease conditions, etc. of the animal is used as an indicator to assess the therapeutic/prophylactic effects of the test compound.

For treating an animal to be test with a test compound, for example, oral administration, intravenous injection, etc. are applied and the treatment is appropriately selected depending upon conditions of the test animal, properties of the test compound, etc. Furthermore, a dose of test compound to be administered can be appropriately chosen depending on method for administration, nature of the test compound, etc.

In screening compounds having the therapeutic effect on, e.g., diabetes mellitus, the non-human mammal deficient in expressing the DNA of the present invention is subjected to a sugar loading treatment, a test compound is administered before or after the sugar loading treatment and, blood sugar level, urinary output, glucose in urine, body weight change, etc. of the animal is measured with passage of time.

The compound obtained using the screening method above is a compound selected from the test compounds described above and exhibits a therapeutic/preventive effect on the diseases caused by deficiencies, damages, etc. of the protein of the present invention. Therefore, the compound can be employed as a safe and low toxic drug for the prevention/treatment of these diseases. Furthermore, compounds derived from the compound obtained by the screening described above can be employed as well.

The compound obtained by the screening method above may be in the form of salts. As such salts, there may be used salts with physiologically acceptable acids (e.g., inorganic acids, organic acids, etc.) or bases (e.g., alkali metal salts, etc.), preferably in the form of physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

A drug comprising the compound or its salt, obtained by the above screening method, may be manufactured in a manner similar to the method for preparing the drug comprising the protein of the present invention described hereinabove.

Since the pharmaceutical preparation thus obtained is safe and low toxic, it can be administered to human or other mammals (e.g., rats, mice, guinea pigs, rabbits, sheep, swine, bovine, horses, cats, dogs, monkeys, etc.).

A dose of the compound or its salt to be administered varies depending upon target disease, subject to be administered, route of administration, etc. For example, when the compound is orally administered, the compound is administered to an adult patient (as 60 kg body weight) with mellitus diabetes generally in a dose of approximately 0.1 to 100 mg, preferably approximately 1.0 to 50 mg, more preferably approximately 1.0 to 20 mg per day. For parenteral administration to an adult patient (as 60 kg body weight) with mellitus diabetes, it is advantageous to administer the compound intravenously in the form of an injectable preparation in a single dose of approximately 0.01 to 30 mg, preferably approximately 0.1 to 20 mg, more preferably approximately 0.1 to 10 mg per day, though the single dosage varies depending upon subject to be administered, target disease, etc. For other animals, the compound can be administered in the corresponding dose with converting it into that for the 60 kg body weight.

### [7b] Method for screening of a compound that promotes or inhibits the activity of a promoter to the DNA of the present invention

The present invention provides a method of screening a compound or its salt that promotes or inhibits the activity of a promoter to the DNA of the present invention, which comprises administering a test compound to a non-human mammal deficient in expressing the DNA of the present invention and detecting expression of the reporter gene.

In the screening method described above, the non-human mammal deficient in expressing the DNA of the present invention is selected from the aforesaid non-human mammal deficient in expressing the DNA of the present invention, as an animal in which the DNA of the present invention is inactivated by introducing a reporter gene and the reporter gene is expressed under control of a promoter to the DNA of the present invention.

The same examples of the test compound apply to those given above.

As the reporter gene, the same specific examples apply. Preferably employed are β-galactosidase (lacZ), soluble alkaline phosphatase gene, luciferase gene and the like.

Since the reporter gene is present under control of a promoter to the DNA of the present invention in the non-human mammal deficient in expressing the DNA of the present invention wherein the DNA of the present invention is substituted with the reporter gene, the activity of the promoter can be detected by tracing expression of a substance encoded by the reporter gene.

For example, when a part of the DNA region encoding the protein of the present invention is substituted with, e.g., β-galactosidase gene (lacZ) derived from Escherichia coli, β-galactosidase is expressed in a tissue where the protein of the present invention should originally be expressed, instead of the protein of the present invention. Thus, the state of expression condition of the protein of the present invention can be readily observed in vivo of an animal by staining with a reagent, e.g., 5-bromo-4-chloro-3-indolyl-β-galactopyranoside (X-gal) which is substrate for β-galactosidase. Specifically, a mouse deficient in the protein of the present invention, or its tissue slice section is fixed with glutaraldehyde, etc. After washing with phosphate buffered saline (PBS), the system is reacted with a staining solution containing X-gal at room temperature or about 37°C for approximately 30 minutes to an hour. After the β-galactosidase reaction is terminated by washing the tissue preparation with 1 mM EDTA/PBS solution, the color formed is observed. Alternatively, mRNA encoding lacZ may be detected in a conventional manner.

The compound or its salts obtained using the aforesaid screening method are compounds selected from the test compounds described above and the compounds that promote or inhibit the activity of a promoter to the DNA of the present invention.

The compound obtained by the screening method above may form salts. As salts of the compound, there may be used salts with physiologically acceptable acids (e.g., inorganic acids, etc.) or bases (e.g., organic acids, etc.), and especially preferred are physiologically acceptable acid addition salts. Examples of such salts are salts with inorganic acids (e.g., hydrochloric acid, phosphoric acid, hydrobromic acid, sulfuric acid, etc.), salts with organic acids (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, etc.) and the like.

The compound or its salt that promotes the promoter activity to the DNA of the present invention can promote expression of the protein of the present invention thereby to promote the function of the protein. Thus, these compounds are useful as safe and low-toxic drugs for the treatment/prevention of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as drugs for the prevention/retardation of aging.

Also, the compound or its salt that inhibits the promoter activity to the DNA of the present invention can inhibit expression of the protein of the present invention to inhibit expression of the protein. Therefore, the compound or its salt is useful as a drug for the prevention/treatment of, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as a drug for the prevention/retardation of aging.

Furthermore, compounds derived from the compound obtained by the screening described above can be employed as well.

The drug containing the compound or its salt obtained by the screening method can be manufactured in a manner similar to the method for manufacturing the drug comprising the protein of the present invention described hereinabove.

Since the pharmaceutical preparation thus obtained is safe and low toxic, it can be administered to human or other mammals (e.g., rats, mice, guinea pigs, rabbits, sheep, swine, bovine, horses, cats, dogs, monkeys, etc.).

A dose of the compound or its salt to be administered varies depending upon target disease, subject to be administered, route of administration, etc. For example, when the compound that promotes the promoter activity to the DNA of the present invention is orally administered, the compound is administered to an adult patient (as 60 kg body weight) with mellitus diabetes generally in a dose of approximately 0.1 to 100 mg, preferably approximately 1.0 to 50 mg, more preferably approximately 1.0 to 20 mg per day. In parenteral administration, a single dose of the compound varies depending on the subject to be administered, target disease, etc., and when the compound that promotes the promoter activity to the DNA of the present invention is administered to an adult patient (as 60 kg body weight) with mellitus diabetes in the form of an injectable preparation, it is advantageous to administer the compound intravenously in a single dose of approximately 0.01 to 30 mg, preferably approximately 0.1 to 20 mg, more preferably approximately 0.1 to 10 mg per day. For other animals, the compound can also be administered in the corresponding dose with converting it into that for the 60 kg body weight.

On the other hand, for example, when the compound that inhibits the promoter activity to the DNA of the present invention is orally administered, the compound is administered to an adult patient (as 60 kg body weight) with mellitus diabetes generally in a dose of approximately 0.1 to 100 mg, preferably approximately 1.0 to 50 mg, more preferably approximately 1.0 to 20 mg per day. In parenteral administration, a single dose of the compound varies depending on the subject to be administered, target disease, etc., and when the compound that inhibits the promoter activity to the DNA of the present invention is administered to an adult patient (as 60 kg body weight) with mellitus diabetes in the form of an injectable preparation, it is advantageous to administer the compound intravenously in a single dose of approximately 0.01 to 30 mg, preferably approximately 0.1 to 20 mg, more preferably approximately 0.1 to 10 mg per day. For other animals, the compound can also be administered in the corresponding dose with converting it into that for the 60 kg body weight.

As described above, the non-human mammal deficient in expressing the DNA of the present invention is extremely useful for screening a compound or its salt that promotes or inhibits the activity of a promoter to the DNA of the present invention, and can thus greatly contribute to investigations of causes for various diseases caused by failure to express the DNA of the present invention or to development of preventive/therapeutic agents for these diseases.

Moreover, when a so-called transgenic animal (gene-transfected animal) is prepared by using a DNA containing the promoter region of the protein of the present invention, ligating genes encoding various proteins downstream the same and injecting the genes into animal oocyte, the polypeptide can be specifically synthesized by the animal so that it becomes possible to investigate the activity in vivo. Furthermore, when an appropriate reporter gene is ligated to the promoter region described above to establish a cell line so as to express the gene, such can be used as a survey system of low molecular weight compounds that specifically promotes or suppresses the ability of producing the protein itself of the present invention in vivo.

In the specification and drawings, the codes of bases, amino acids, etc. are denoted in accordance with the IUPAC-IUB Commission on Biochemical Nomenclature or by the common codes in the art, examples of which are shown below. For amino acids that may have the optical isomer, L form is presented unless otherwise indicated.
- DNA: : deoxyribonucleic acid
- cDNA: : complementary deoxyribonucleic acid
- A: : adenine
- T: : thymine
- G: : guanine
- C: : cytosine
- RNA: : ribonucleic acid
- mRNA: : messenger ribonucleic acid
- dATP: : deoxyadenosine triphosphate
- dTTP :: deoxythymidine triphosphate
- dGTP: : deoxyguanosine triphosphate
- dCTP: : deoxycytidine triphosphate
- ATP: : adenosine triphosphate
- EDTA: : ethylenediaminetetraacetic acid
- SDS: : sodium dodecyl sulfate
- Gly: : glycine
- Ala: : alanine
- Val: : valine
- Leu: leucine
- Ile: : isoleucine
- Ser: : serine
- Thr: : threonine
- Cys: : cysteine
- Met: : methionine
- Glu: : glutamic acid
- Asp: : aspartic acid
- Lys: : lysine
- Arg: : arginine
- His: : histidine
- Phe: : phenylalanine
- Tyr: : tyrosine
- Trp: : tryptophan
- Pro: : proline
- Asn: : asparagine
- Gln: : glutamine
- pGlu: : pyroglutamic acid

The substituents, protective groups and reagents, which are frequently used throughout the specification, are shown by the following abbreviations.
- Me: : methyl
- Et: : ethyl
- Bu: : butyl
- Ph: : phenyl
- TC: : thiazolidine-4(R)-carboxamide
- Tos: : p-toluenesulfonyl
- CHO :: formyl
- Bzl: : benzyl
- Cl₂-Bzl: : 2,6-dichlorobenzyl
- Bom: : benzyloxymethyl
- Z: : benzyloxycarbonyl
- Cl-Z: : 2-chlorobenzyloxycarbonyl
- Br-Z: : 2-bromobenzyloxycarbonyl
- Boc: : t-butoxycarbonyl
- DNP: :dinitrophenol
- Trt: : trityl
- Bum: : t-butoxymethyl
- Fmoc: : N-9-fluorenylmethoxycarbonyl
- HOBt: 1-hydroxybenztriazole
- HOOBt: : 3,4-dihydro-3-hydroxy-4-oxo-1,2,3-benzotriazine
- HONB: : 1-hydroxy-5-norbornene-2,3-dicarboximide
- DCC: : N,N'-dicyclohexylcarbodiimide

The sequence identification numbers in the sequence listing of the specification indicates the following sequence, respectively.

### [SEQ ID NO:1]

This shows the amino acid sequence of 568 amino acids for human TCH169 protein obtained in EXAMPLE 1.

### [SEQ ID NO:2]

This shows the base sequence of DNA encoding human TCH169 protein having the amino acid sequence represented by SEQ ID NO: 1.

### [SEQ ID NO: 3]

This shows the base sequence of primer A 10 used in EXAMPLE 2.

### [SEQ ID NO: 4]

This shows the base sequence of primer B9 used in EXAMPLE 2.

### [SEQ ID NO: 5]

This shows the base sequence of TaqMan probe T1 used in EXAMPLE 2.

### [SEQ ID NO: 6]

This shows the base sequence of primer A7 used in EXAMPLE 1.

### [SEQ ID NO: 7]

This shows the base sequence of primer B6 used in EXAMPLE 1.

### [SEQ ID NO: 8]

This shows the base sequence of primer SP6 used in EXAMPLES 1 and 4.

### [SEQ ID NO: 9]

This shows the base sequence of primer T7 used in EXAMPLES 1 and 4.

### [SEQ ID NO: 10]

This shows the base sequence of primer A1 used in EXAMPLE 1.

### [SEQ ID NO: 11]

This shows the base sequence of primer A2 used in EXAMPLE 1.

### [SEQ ID NO: 12]

This shows the base sequence of primer F1 used in EXAMPLE 1.

### [SEQ ID NO: 13]

This shows the base sequence of primer F2 used in EXAMPLE 1.

### [SEQ ID NO: 14]

This shows the base sequence of primer F3 used in EXAMPLE 1.

### [SEQ ID NO: 15]

This shows the base sequence of primer B I used in EXAMPLE 1.

### [SEQ ID NO: 16]

This shows the base sequence of primer B2 used in EXAMPLE 1.

### [SEQ ID NO: 17]

This shows the base sequence of primer B8 used in EXAMPLE 1.

### [SEQ ID NO: 18]

This shows the base sequence of primer R1 used in EXAMPLE 1.

### [SEQ ID NO: 19]

This shows the base sequence of primer R2 used in EXAMPLE 1.

### [SEQ ID NO: 20]

This shows the base sequence of primer R3 used in EXAMPLE 1.

### [SEQ ID NO: 21]

This shows the base sequence of primer R4 used in EXAMPLE 1.

### [SEQ ID NO: 22]

This shows the base sequence of cDNA containing the full-length human TCH169 gene acquired in EXAMPLE 1.

### [SEQ ID NO: 23]

This shows the amino acid sequence of 572 amino acids for mouse TCH169 acquired in EXAMPLE 4.

### [SEQ ID NO: 24]

This shows the base sequence of DNA encoding mouse TCH169 protein having the amino acid sequence represented by SEQ ID NO: 23.

### [SEQ ID NO: 25]

This shows the base sequence of primer AP1 used in EXAMPLE 3.

### [SEQ ID NO: 26]

This shows the base sequence of primer mB5 used in EXAMPLE 3.

### [SEQ ID NO:27]

This shows the base sequence of primer AP2 used in EXAMPLE 3.

### [SEQ ID NO: 28]

This shows the base sequence of primer mB3 used in EXAMPLE 3.

### [SEQ ID NO: 29]

This shows the base sequence of primer mA4 used in EXAMPLE 4.

### [SEQ ID NO: 30]

This shows the base sequence of primer mB6 used in EXAMPLE 4.

### [SEQ ID NO: 31]

This shows the base sequence of primer mA1 used in EXAMPLE 4.

### [SEQ ID NO: 32]

This shows the base sequence of primer mA2 used in EXAMPLE 4.

### [SEQ ID NO: 33]

This shows the base sequence of primer mB2 used in EXAMPLE 4.

### [SEQ ID NO: 34]

This shows the base sequence of primer mR1 used in EXAMPLE 4.

### [SEQ ID NO: 35]

This shows the base sequence of 5' upstream cDNA of mouse TCH169 acquired in EXAMPLE 3.

### [SEQ ID NO: 36]

This shows the base sequence of cDNA containing the full-length mouse TCH169 gene obtained in EXAMPLE 4.

### [SEQ ID NO: 37]

This shows the base sequence of primer mTF used in EXAMPLE 5.

### [SEQ ID NO: 38]

This shows the base sequence of primer mTR used in EXAMPLE 5.

### [SEQ ID NO: 39]

This shows the base sequence of TaqMan probe mT1 used in EXAMPLE 5.

### [SEQ ID NO: 40]

This shows the base sequence of primer OF used in EXAMPLE 6.

### [SEQ ID NO: 41]

This shows the base sequence of primer OR used in EXAMPLE 6.

### [SEQ ID NO: 42]

This shows the base sequence of primer BGH RV used in EXAMPLE 6.

The transformant Escherichia coli TOP10/pCR-BIuntII-TCH169 obtained in EXAMPLE 1 described below has been on deposit since September 13, 2001 with International Patent Organisms Depository, National Institute of Advanced Industrial Science and Technology, located at Central 6, 1-1-1 Higashi, Tsukuba, Ibaraki, Japan, as the Accession Number FERM BP-7734 and since August 29, 2001 with Institute for Fermentation (IFO), located at 2-17-85 Juso-honmachi, Yodogawa-ku, Osaka-shi, Osaka, Japan, as the Accession Number IFO 16690.

The transformant Escherichia coli TOP10/pCR-BluntII-mTCH169 obtained in EXAMPLE 4 described below has been on deposit since April 10, 2002 with International Patent Organisms Depository, National Institute of Advanced Industrial Science and Technology, located at Central 6, 1-1-1 Higashi, Tsukuba, Ibaraki, Japan, as the Accession Number FERM BP-8010 and since April 2, 2002 with Institute for Fermentation (IFO), located at 2-17-85 Juso-honmachi, Yodogawa-ku, Osaka-shi, Osaka, Japan, as the Accession Number IFO 16785.

### EXAMPLES

Hereinafter the present invention is described in more detail with reference to EXAMPLES, but is not deemed to limit the scope of the present invention thereto. The gene manipulation procedures using Escherichia coli were performed according to the methods described in the Molecular Cloning.

### EXAMPLE 1:

### Cloning of human TCH169 gene cDNA

Using two primer DNAs, i.e., primer A7 (SEQ ID NO: 6) and primer B6 (SEQ ID NO: 7), PCR was performed on human liver cDNA library (manufactured by Takara Shuzo Co., Ltd.) using Pyrobest DNA Polymerase (manufactured by Takara Shuzo Co., Ltd.) to obtain two clones #1 and #2 from plasmid clone pCR-BluntII-TCH169. The clones were reacted using a primer DNA [primer SP6 (SEQ ID NO:8), primer T7(SEQ ID NO:9), primer A1(SEQ ID NO:10) , primer A2(SEQ ID NO:11), primer F1(SEQ ID NO:12), primer F2(SEQ ID NO:13), primer F3(SEQ ID NO:14) , primer B1(SEQ ID NO:15), primer B2(SEQ ID NO:16), primer B8(SEQ ID NO: 17), primer R1(SEQ ID NO: 18), primer R2(SEQ ID NO: 19), primer R3(SEQ ID NO:20), primer R4(SEQ ID NO:21)] and a BigDye Terminator Cycle Sequencing Kit (manufactured by Applied Biosystems, Inc.), and the base sequence of the insert cDNA fragment was determined on a DNA sequencer, ABI PRISM 3100 DNA Analyzer (manufactured by Applied Biosystems, Inc.). As a result, the two clones were found to contain the same DNA fragment and have the 2320 base sequence (SEQ ID NO: 22). The cDNA fragment (SEQ ID NO:2) encoded the 568 amino acid sequence (SEQ ID NO:1), and the protein having the amino acid sequence was named human TCH169 protein.

The transformant bearing a plasmid containing the cDNA fragment was named Escherichia coli TOP10/pCR-BluntII-TCH169.

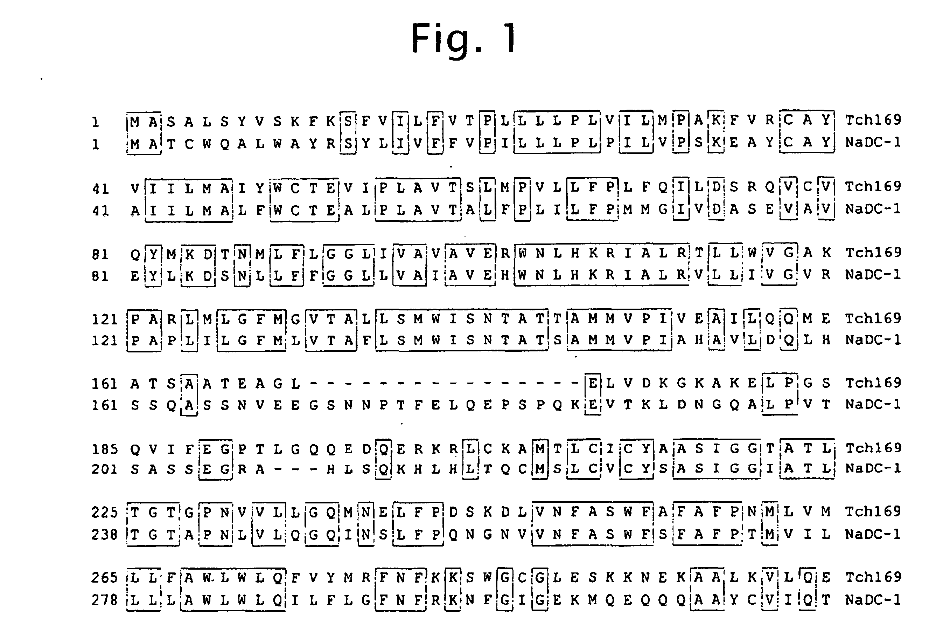

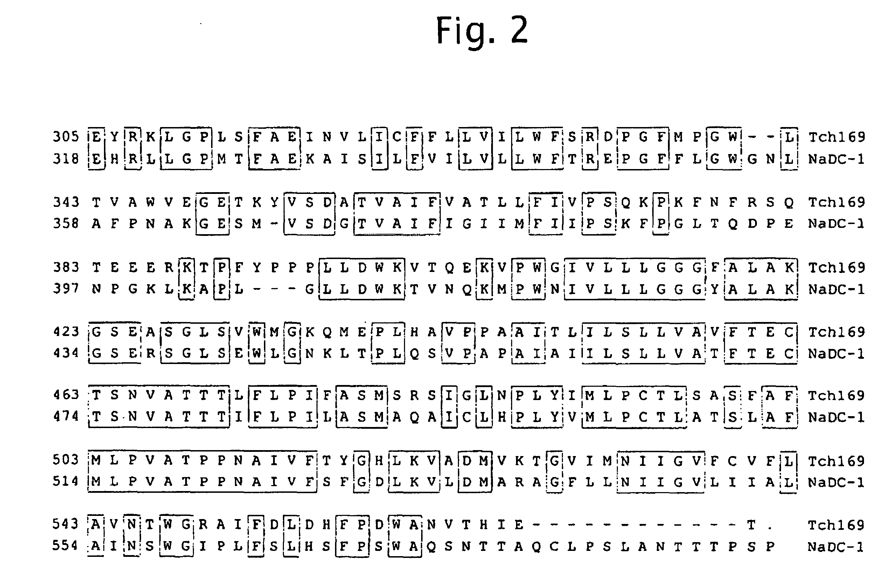

Based on homology search with Owl using Blast P [Nucleic Acids Res., 25, 3389 (1997)], the cDNA was found to be a novel gene classified in a dicarboxylic acid transporter (FIGS. 1 and 2). The cDNA showed 65% homology on a base level and 56% homology on an amino acid level, to sodium-dependent dicarboxylate transporter NaDCl [Am. J. Physiol., 270, F642 (1996)] reported in human.

### EXAMPLE 2:

### Analysis of tissue distribution of the human TCH169 gene product

Using two primer DNAs, i.e., primer A 10 (SEQ ID NO:3) and primer B9 (SEQ ID NO:4) designed based on the sequence of TCH169 as well as TaqMan probe T1 (SEQ ID NO:5), the expression level of TCH169 in cDNAs (Human MTC panel I and Human MTC panel II: manufactured by Clontech, Inc.) in each of the human organs (heart, brain, placenta, lung, liver, skeletal muscle, kidney, pancreas, spleen, thymus, prostate, testis, ovary, small intestine, colon and peripheral leukocyte) was assayed by TaqMan PCR. Using TaqMan Universal PCR Master Mix (manufactured by Applied Biosystems, Inc.), the reaction was carried out by initially reacting at 50°C for 2 minutes, then maintaining at 95°C for 10 minutes, and repeating 40 cycles of one reaction set to include at 95°C for 15 seconds and at 60°C for 1 second, while detection was simultaneously made on the ABI PRISM 7900 sequence detection system (manufactured by Applied Biosystems, Inc.).

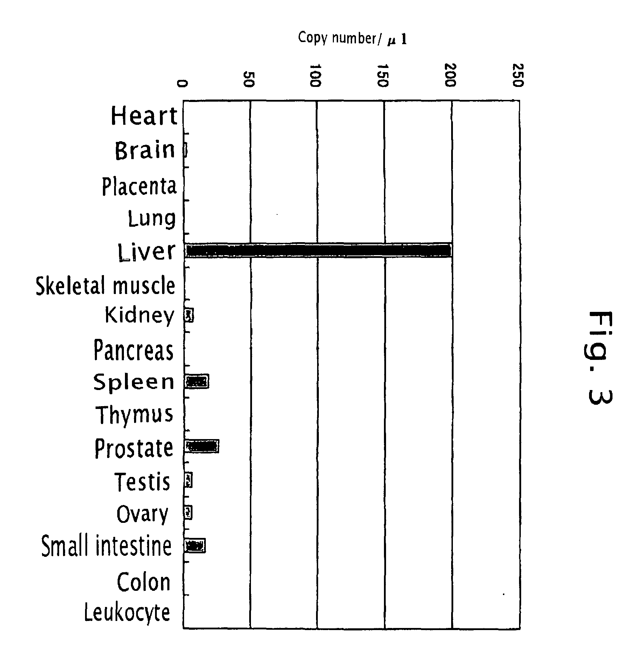

The results are shown in FIG. 3. The human TCH169 gene product (mRNA) was expressed slightly in the kidney, testis and ovary and somewhat in the prostate, spleen and small intestine. The most potent expression was found in the liver.

### EXAMPLE 3:

### Cloning of mouse TCH169 protein-encoding cDNA at the 5' upstream end

A base sequence at the 5' upstream end of cDNA encoding mouse TCH169 protein was clarified by the 5' RACE method.

Using two primer DNAs, i.e., primer AP1 (SEQ ID NO:25) and primer mB5 (SEQ ID NO:26), primary PCR was carried out on mouse brain Marathon-Ready cDNA (manufactured by Clontech Inc.) under the conditions (1) to (4) below, using Advantage 2 DNA Polymerase (manufactured by Clontech Inc.).
(1) 94°C for 30 seconds
(2) 5 cycles of one set to include 94°C for 5 seconds - 72°C for 4 minutes
(3) 5 cycles of one set to include 94°C for 5 seconds - 70°C for 4 minutes
(4) 25 cycles of one set to include 94°C for 5 seconds - 68°C for 4 minutes.
   Further using the product of this primary PCR as a template and using primer AP2 (SEQ ID NO:27) and primer mB3 (SEQ ID NO:28), nested PCR was carried out under the conditions (5) to (7) using Advantage 2 DNA Polymerase (manufactured by Clontech Inc.).
(5) 94°C for 30 seconds
(6) 5 cycles of one set to include 94°C for 5 seconds - 70°C for 4 minutes
(7) 30 cycles of one set to include 94°C for 5 seconds - 68°C for 4 minutes.

After the nested PCR reaction solution was subjected to gel electrophoresis, the major band was purified. The PCR product was reacted using primer mB3 (SEQ ID NO:28) and a BigDye Terminator Cycle Sequencing Kit (manufactured by Applied Biosystems, Inc.), and the base sequence of the amplified DNA fragment was determined on a DNA sequencer, ABI PRISM 3100 DNA Analyzer (manufactured by Applied Biosystems, Inc.). As a result, the base sequence represented by SEQ ID NO: 35 was obtained.

### EXAMPLE 4:

### Cloning of cDNA encoding mouse TCH169 protein

Using two primer DNAs, i.e., primer mA4 (SEQ ID NO:29) and primer mB6 (SEQ ID NO:30), PCR was carried out on mouse brain Marathon-Ready cDNA (manufactured by Clontech Inc.) under the conditions (1) to (3) below, using Pyrobest DNA Polymerase(manufactured by Takara Shuzo Co., Ltd.).
(1) 94°C for 2 minutes
(2) 30 cycles of one set to include 98°C for 10 seconds - 68°C for 30 seconds - 72°C for 3.5 minutes
(3) 72°C for 10 minutes.

The amplified product thus obtained was cloned using Zero Blunt TOPO Cloning Kit (manufactured by Invitrogen, Inc.) to acquire plasmid pCR-BluntII-mTCH169.

Using a primer DNA [primer SP6 (SEQ ID NO:8), primer T7 (SEQ ID NO:9), primer mA1 (SEQ ID NO:31), primer mA2 (SEQ ID NO:32), primer mB2 (SEQ ID NO:33), primer mR1 (SEQ ID NO:34)] and a BigDye Terminator Cycle Sequencing Kit (manufactured by Applied Biosystems, Inc.), the reaction was carried out and the base sequence of the insert cDNA fragment was determined on a DNA sequencer, ABI PRISM 3100 DNA Analyzer (manufactured by Applied Biosystems, Inc.). As a result, the clone was found to have the 1826 base sequence (SEQ ID NO: 36). The cDNA fragment (SEQ ID NO: 24) encoded the 572 amino acid sequence (SEQ ID NO: 23), and the protein having the said amino acid sequence was named mouse TCH169 protein.

The transformant bearing the plasmid containing the cDNA fragment was named Escherichia coli TOP10/pCR-BluntII-mTCH169.

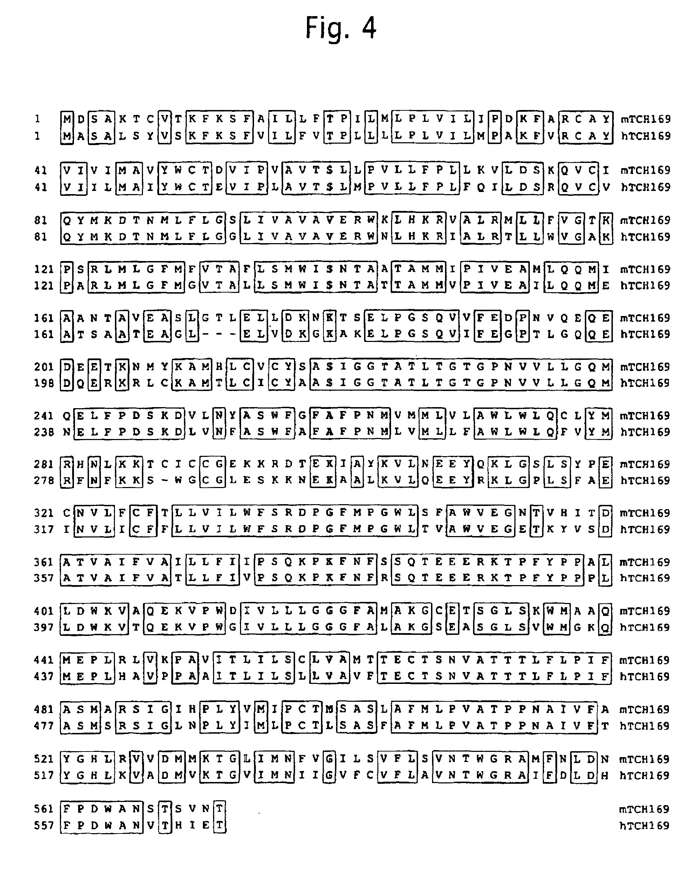

Based on homology search with Owl using Blast P [Nucleic Acids Res., 25, 3389, 1997], the cDNA was found to be a mouse ortholog of a novel gene, human TCH169 classified into a sodium-dependent dicarboxylate transporter family (FIG. 4). The mouth TCH169 showed 77% homology on a base level and 74% homology on an amino acid level, to human TCH169, and 50% homology on a base level and 48% homology on an amino acid level, to sodium-dependent dicarboxylate transporter NaDCl [Am. J. Physiol., 279, F482 (2000)] reported in mouse.

### EXAMPLE 5:

### Analysis of tissue distribution of the mouse TCH169 gene product

Using two primer DNAs, i.e., primer mTF (SEQ ID NO:37) and primer mTR (SEQ ID NO:38) designed based on the sequence of mouse TCH169 as well as TaqMan probe mT1 (SEQ ID NO: 39), the expression level of TCH169 in cDNAs (Mouse MTC panel I and Mouse MTC panel II: manufactured by Clontech, Inc.) in each of the mouse organs (bone marrow, eye, lymph node, smooth muscle, prostate, thymus, stomach, uterus, heart, brain, spleen, lung, liver, skeletal muscle, kidney, testis, embryo (7 days), embryo (11 days), embryo (15 days), embryo (17 days)) was assayed by TaqMan PCR. Using TaqMan Universal PCR Master Mix (manufactured by Applied Biosystems, Inc.), the reaction was carried out by initially reacting at 50°C for 2 minutes, maintaining at 95°C for 10 minutes and then repeating 40 cycles of one reaction set to include at 95°C for 15 seconds and at 60°C for 1 second, while detection was simultaneously made on the ABI PRISM 7900 sequence detection system (manufactured by Applied Biosystems, Inc.).

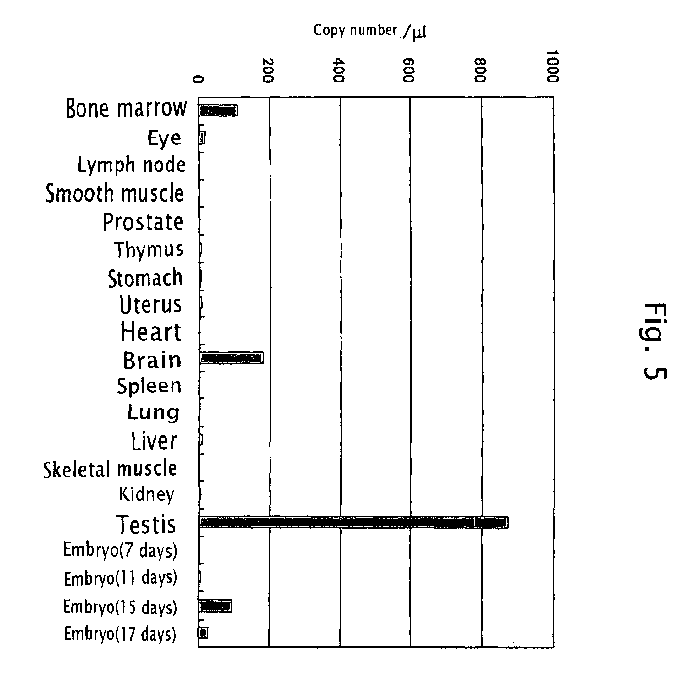

The results are shown in FIG. 5. The mouse TCH169 gene product (mRNA) was expressed slightly in the thymus, stomach, uterus, liver, kidney and embryo (11 days) and somewhat in the eye and embryo (17 days). The most potent expression was found in the testis.

### EXAMPLE 6:

### Construction of human TCH169 expression vector

Human TCH169 (SEQ ID NO: 1) expression vector was constructed by the following procedure. Using as a template 10 ng of the plasmid obtained in EXAMPLE 1 and using primer OF (SEQ ID NO:40) and primer OR (SEQ ID NO:41), PCR was carried out under the conditions (1) to (3) below, using Pyrobest DNA Polymerase(manufactured by Takara Shuzo Co., Ltd.). For cloning to the vector, the 5' end primer OF and the 3' end primer OR were designed to add the Hind III site and the Xba I site, respectively, at the 5' end.
(1) 94°C for 2 minutes
(2) 30 cycles of one set to include 98°C for 10 seconds - 65°C for 30 seconds - 72°C for 3.5 minutes
(3) 72°C for 10 minutes.

After the PCR reaction solution was subjected to gel electrophoresis, the major band was purified. The PCR fragment thus obtained was digested with restriction enzymes Hind III and Xba I by maintaining at 37°C for an hour. The reaction solution was subjected to gel electrophoresis and then purified. The purified product was ligated to animal cell expression vector pcDNA3.1 (+) (manufactured by Invitrogen, Inc.) at the Hind III site and the Xba I site, using Takara ligation kit ver.2 (manufactured by Takara Shuzo Co., Ltd.). After ethanol precipitation of the ligation solution, transfection was made on competent cells (Escherichia coli) TOP 10 (manufactured by Invitrogen, Inc.). From a plurality of colonies a plasmid was prepared, and its base sequence was reacted using a primer DNA [primer BGH RV (SEQ ID NO:42), primer T7 (SEQ ID NO:9), primer A2 (SEQ ID NO: 11), primer F1 (SEQ ID NO: 12), primer F2 (SEQ ID NO:13), primer B1 (SEQ ID NO:15), primer B2 (SEQ ID NO:16), primer R1 (SEQ ID NO:18), primer R2 (SEQ ID NO:19)] and a BigDye Terminator Cycle Sequencing Kit (manufactured by Applied Biosystems, Inc.), which was applied on a DNA sequencer, ABI PRISM 3100 DNA Analyzer (manufactured by Applied Biosystems, Inc.) to confirm the base sequence. The transformant bearing this plasmid was named Escherichia coli TOP10/pCDNA3.1 (+)-TCH169.

### EXAMPLE 7:

### Production of the TCH169 expression CHO cell line and assay for the expression level of transfected gene

TOP10/pCDNA3.1 (+)-TCH169 was cultured and plasmid DNA was prepared from the cells of this Escherichia coli using EndoFree Plasmid Maxi Kit (manufactured by Qiagen). This plasmid DNA was transfected to CHO dhfr⁻ cells using FuGENE 6 Transfection Reagent (manufactured by Roche) in accordance with the protocols attached thereto. A mixture of 2 µg of the plasmid DNA and a transfection reagent was poured into a Petri dish of 6 cm diameter, in which 3 x 10⁶ CHO dhfr⁻ cells had been seeded 24 hours before the transfection. After incubation for a day in MEMa medium supplemented with 10% fetal calf serum, the cells were stripped by treatment with trypsin and the recovered cells were seeded on a 96 well plate in 10-50 cells per well. After further 24 hours, 0.5 mg/ml of G418 was supplemented to the medium. For 7 days thereafter, TCH169 expression cells were selected in the medium containing 0.5-1.0 mg/ml of G418. Regarding 10 wells in which 1 to 3 colonies per well were found to grow, the cells were incubated in a 6 well plate and the total RNA was prepared from the grown cells using RNeasy Mini Kit or RNeasy 96 Kit (both manufactured by Qiagen). Reverse transcription of the total RNA prepared was performed using TaqMan Reverse Transcription Reagents (manufactured by Applied Biosystems, Inc.) to prepare cDNA. In the cDNA, the expression level of TCH169 was determined by TaqMan PCR using primer A10 (SEQ ID NO: 3) and primer B9 (SEQ ID NO: 4) as well as TaqMan Probe T1 (SEQ ID NO: 5) used in EXAMPLE 2. The reaction was carried out using TaqMan Universal PCR Master Mix (manufactured by Applied Biosystems, Inc.) initially at 50°C for 2 minutes and further at 95°C for 10 minutes, and then by repeating 40 cycles of one reaction cycle set to include 95°C for 15 seconds and 60°C for 1 minute, while detection was simultaneously made on ABI PRISM 7900 sequence detection system (manufactured by Applied Biosystems, Inc.). Clone Nos. 2 and 13 were selected as the human TCH169 gene expression cell line on a high level (polyclonal). These clones were seeded on a 96 well plate in 0.5 cells per well and cultured in a G418-supplemented medium for 7 to 10 days to acquire monoclonal clone. From the clone, the total RNA was prepared and the expression level of human TCH169 gene was determined by TaqMan PCR. Clone No. 2-11 was selected as the human TCH169 gene expression cell line on a high level (monoclonal).

### EXAMPLE 8:

### Assay for [1,5-¹⁴C] citric acid uptake in the human TCH169 gene expression cell line

Using human TCH169 gene expression cell line clone No. 2-11 acquired in EXAMPLE 7, the uptake of [1,5-¹⁴C] citric acid was assayed. Human TCH169 gene expression cell line clone No. 2-11 was seeded on a 96 well plate in 4 x 10⁴ cells per well, followed by incubation at 37°C for 24 hours. After the medium was removed, the cells were washed 3 times with 150 µL NMDG buffer (140 mM N-methyl-D(-)-glucamine, 25 mM Hepes/Tris, 5.4 mM KCI, 1.8 mM CaCl₂, 0.8 mM MgSO₄, 5 mM glucose, pH 7.4-7.6), and 150 µL NMDG buffer was supplemented thereto followed by incubation at 37°C for an hour. After the buffer was replaced with 90 µL NaCl buffer (140 mM NaCl, 25 mM Hepes/Tris, 5.4 mM KCI, 1.8 mM CaCl₂, 0.8 mM MgSO₄, 5 mM glucose, pH 7.4-7.6) or 90 µL NMDG buffer, 10 µL of 223 µM [1,5-¹⁴C] citric acid (manufactured by Amersham-Pharmacia-Biotech) was added and incubation was performed at 37°C for 15 minutes, 30 minutes and an hour followed by washing 3 times with 200 µL PBS (manufactured by Takara Shuzo Co., Ltd.). After 100 µL of Opitiphase 'SuperMix' (manufactured by Perkin-Elmer-Life Science) was added to the system and the mixture was stirred, the amount of [1,5-¹⁴C] citric acid taken up into the cells was measured in terms of the radioactivity. Measurement was performed with 1450 MICROBETA PLUS LIQUID SCINTILLATION COUNTER (manufactured by Perkin-Elmer-Life Science). CHO dhfr⁻ cells transfected with vector pcDNA3.1(+) (Mock) was also treated in a similar manner to measure the radioactivity as well.

The results are shown in FIGS. 6 and 7. The results reveal that [1,5-¹⁴C] citric acid is taken up into the human TCH169 expression CHO cells in the presence of 140 mM NaCl.

### INDUSTRIAL APPLICABILITY

The protein of the present invention is useful as a diagnostic marker for, e.g., hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.), or for aging. The compound that promotes or inhibits the activity of the protein obtained by the screening method using the protein is useful as an agent for the prevention/treatment of, for example, hepatic diseases (e.g., liver cirrhosis, hepatitis, alcoholic liver disease, etc.), prostate disorders (e.g., prostatic hyperplasia, prostatitis, etc.), splenic diseases (e.g., hypersplenism, splenomegalic syndrome, etc.), renal diseases (e.g., nephritis, renal failure, glomerulonephritis, diabetic nephropathy, focal glomerular sclerosis, nephrotic syndrome, renal edema, etc.), metabolic diseases (e.g., mellitus diabetes, hyperlipemia, etc.), cardiovascular diseases (e.g., arteriosclerosis, etc.) or cancers (e.g., lung cancer, renal cancer, hepatic cancer, non-small cell lung cancer, prostate cancer, gastric cancer, bladder cancer, breast cancer, cervical cancer, colon cancer, rectal cancer, pancreatic cancer, etc.) or the like, or as an agent for the prevention/retardation of aging.

## Claims

1. A protein comprising the same or substantially the same amino acid sequence as the amino acid sequence represented by SEQ ID NO: 1 or SEQ ID NO: 23, or a salt thereof.

2. A protein consisting of the amino acid sequence represented by SEQ ID NO: 1, or a salt thereof.

3. A protein consisting of the amino acid sequence represented by SEQ ID NO: 23, or a salt thereof.

4. A partial peptide of the protein according to claim 1, or a salt thereof.

5. A polynucleotide containing a polynucleotide encoding the protein according to claim 1 or the partial peptide according to claim 4.

6. A polynucleotide according to claim 5, which is DNA.

7. A polynucleotide consisting of the base sequence represented by SEQ ID NO: 2 or SEQ ID NO: 22.

8. A polynucleotide consisting of the base sequence represented by SEQ ID NO: 24 or SEQ ID NO: 36.

9. A recombinant vector containing the polynucleotide according to claim 5.

10. A transformant transformed with the recombinant vector according to claim 9.

11. A method of manufacturing the protein according to claim 1 or the partial peptide according to claim 4, or a salt thereof, which comprises culturing the transformant according to claim 10, producing, accumulating and collecting the protein according to claim 1 or the partial peptide according to claim 4.

12. A drug comprising the protein according to claim 1 or the partial peptide according to claim 4, or a salt of said protein or partial peptide.

13. A drug comprising the polynucleotide according to claim 5.

14. A diagnostic agent comprising the polynucleotide according to claim 5.

15. An antibody to the protein according to claim 1 or the partial peptide according to claim 4, or a salt of said protein or partial peptide.

16. A drug comprising the antibody according to claim 15.

17. A diagnostic agent comprising the antibody according to claim 15.

18. A polynucleotide comprising a base sequence complementary or substantially complementary to the polynucleotide according to claim 5 or a part of the base sequence.

19. A drug comprising the polynucleotide according to claim 18.

20. A method of screening a compound or its salt that promotes or inhibits the activity of the protein according to claim 1 or the partial peptide according to claim 4, or a salt thereof, which comprises using the protein according to claim 1 or the partial peptide according to claim 4, or a salt of said protein or partial peptide.

21. A kit for screening a compound or its salt that promotes or inhibits the activity of the protein according to claim 1 or the partial peptide according to claim 4, or a salt thereof, comprising the protein according to claim 1 or the partial peptide according to claim 4, or a salt of said protein or partial peptide.

22. A compound or its salt that promotes or inhibits the activity of the protein according to claim 1 or the partial peptide according to claim 4, or a salt thereof, which is obtainable using the screening method according to claim 20 or the screening kit according to claim 21.

23. A drug comprising the compound or its salt according to claim 22.

24. A method of screening a compound or its salt that promotes or inhibits expression of a gene for the protein according to claim 1, which comprises using the polynucleotide according to claim 5.

25. A kit for screening a compound or its salt that promotes or inhibits expression of a gene for the protein according to claim 1, comprising the polynucleotide according to claim 5.

26. A compound or its salt that promotes or inhibits expression of a gene for the protein according to claim 1, which is obtainable using the screening method according to claim 24 or the screening kit according to claim 25.

27. A drug comprising the compound or its salt according to claim 26.

28. A method of quantifying the protein according to claim 1, which comprises using the antibody according to claim 15.

29. A diagnostic method for a disease associated with functions of the protein according to claim 1, which comprises using the quantification method according to claim 28.

30. A method of screening a compound or its salt that promotes or inhibits expression of the protein according to claim 1, which comprises using the antibody according to claim 15.

31. A kit for screening a compound or its salt that promotes or inhibits expression of the protein according to claim 1, comprising the antibody according to claim 15.

32. A compound or its salt that promotes or inhibits expression of the protein according to claim 1, which is obtainable using the screening method according to claim 30 or the screening kit according to claim 31.

33. A drug comprising the compound or its salt according to claim 32.

34. A drug according to claim 13, 16, 19, 23, 27 or 33, which is an agent for the prevention/treatment of hepatic dysfunctions, prostate diseases, splenic diseases, renal diseases, metabolic diseases, circulatory disorders or cancer, or an agent for prevention/retardation of aging.

35. A diagnostic agent according to claim 14 or 17, which is a diagnostic agent for hepatic dysfunctions, prostate diseases, splenic diseases, renal diseases, metabolic diseases, circulatory disorders or cancer.

36. A method for the prevention/treatment of hepatic dysfunctions, prostate diseases, splenic diseases, renal diseases, metabolic diseases, circulatory disorders or cancer, or for the prevention/retardation of aging, which comprises administering an effective dose of a compound or its salt according to claim 22, 26 or 32 to a mammal.

37. Use of a compound or its salt according to claim 22, 26 or 32 for manufacturing an agent for the prevention/treatment of hepatic dysfunctions, prostate diseases, splenic diseases, renal diseases, metabolic diseases, circulatory disorders or cancer, or for the prevention/retardation of aging.


SEQUENCE LISTING

<110> Takeda Chemical Industries, Ltd.

<120> Novel Protein and its DNA

<130> P02-0109PCT

<150> JP 2001-281992
<151> 2001-09-17

<150> JP 2001-306873
<151> 2001-10-02

<150> JP 2002-113279
<151> 2002-04-16

<160> 42

<210> 1
<211> 568
<212> PRT
<213> Homo sapiens

<400> 1

<210> 2
<211> 1704
<212> DNA
<213> Homo sapiens

<400> 2
atggcctcgg cgctgagcta tgtctccaag ttcaagtcct tcgtgatctt gttcgtcacc 60
ccgctcctgc tgctgccact cgtcattctg atgcccgcca agtttgtcag gtgtgcctac 120
gtcatcatcc tcatggccat ttactggtgc acagaagtca tccctctggc tgtcacctct 180
ctcatgcctg tcttgctttt cccactcttc cagattctgg actccaggca ggtgtgtgtc 240
cagtacatga aggacaccaa catgctgttc ctgggcggcc tcatcgtggc cgtggctgtg 300
gagcgctgga acctgcacaa gaggatcgcc ctgcgcacgc tcctctgggt gggggccaag 360
cctgcacggc tgatgctggg cttcatgggc gtcacagccc tcctgtccat gtggatcagt 420
aacacggcaa ccacggccat gatggtgccc atcgtggagg ccatattgca gcagatggaa 480
gccacaagcg cagccaccga ggccggcctg gagctggtgg acaagggcaa ggccaaggag 540
ctgccaggga gtcaagtgat ttttgaaggc cccactctgg ggcagcagga agaccaagag 600
cggaagaggt tgtgtaaggc catgaccctg tgcatctgct acgcggccag catcgggggc 660
accgccaccc tgaccgggac gggacccaac gtggtgctcc tgggccagat gaacgagttg 720
tttcctgaca gcaaggacct cgtgaacttt gcttcctggt ttgcatttgc ctttcccaac 780
atgctggtga tgctgctgtt cgcctggctg tggctccagt ttgtttacat gagattcaat 840
tttaaaaagt cctggggctg cgggctagag agcaagaaaa acgagaaggc tgccctcaag 900
gtgctgcagg aggagtaccg gaagctgggg cccttgtcct tcgcggagat caacgtgctg 960
atctgcttct tcctgctggt catcctgtgg ttctcccgag accccggctt catgcccggc 1020
tggctgactg ttgcctgggt ggagggtgag acaaagtatg tctccgatgc cactgtggcc 1080
atctttgtgg ccaccctgct attcattgtg ccttcacaga agcccaagtt taacttccgc 1140
agccagactg aggaagaaag gaaaactcca ttttatcccc ctcccctgct ggattggaag 1200
gtaacccagg agaaagtgcc ctggggcatc gtgctgctac tagggggcgg atttgctctg 1260
gctaaaggat ccgaggcctc ggggctgtcc gtgtggatgg ggaagcagat ggagcccttg 1320
cacgcagtgc ccccggcagc catcaccttg atcttgtcct tgctcgttgc cgtgttcact 1380
gagtgcacaa gcaacgtggc caccaccacc ttgttcctgc ccatctttgc ctccatgtct 1440
cgctccatcg gcctcaatcc gctgtacatc atgctgccct gtaccctgag tgcctccttt 1500
gccttcatgt tgcctgtggc caccccctca aatgccatcg tgttcaccta tgggcacctc 1560
aaggttgctg acatggtgaa aacaggagtc ataatgaaca taattggagt cttctgtgtg 1620
tttttggctg tcaacacctg gggacgggcc atatttgact tggatcattt ccctgactgg 1680
gctaatgtga cacatattga gact 1704

<210> 3
<211> 21
<212> DNA
<213> Artificial Sequence

<220>
<223> Primer A10 for amplifying homo sapiens TCH169 gene

<400> 3
ggcaggtgtg tgtccagtac a                    21

<210> 4
<211> 16
<212> DNA
<213> Artificial Sequence

<220>
<223> Primer B9 for amplifying homo sapiens TCH169 gene

<400> 4
cagccacggc cacgat                          16

<210> 5
<211> 22
<212> DNA
<213> Artificial Sequence

<220>
<223> TaqMan Probe T1

<400> 5
acatgctgtt cctgggcggc ct                      22

<210> 6
<211> 25
<212> DNA
<213> Artificial Sequence

<220>
<223> Primer A7 for amplifying homo sapiens TCH169 gene

<400> 6
gcctctagct gcccctcact cgtct                25

<210> 7
<211> 25
<212> DNA
<213> Artificial Sequence

<220>
<223> Primer B6 for amplifying homo sapiens TCH169 gene

<400> 7
aggctctgat tcccactcgc actcc                25

<210> 8
<211> 18
<212> DNA
<213> Artificial Sequence

<220>
<223> Primer SP6 for sequencing

<400> 8
atttaggtga cactatag                        18

<210> 9
<211> 19

<212> DNA
<213> Artificial Sequence

<220>
<223> Primer T7 for sequencing

<400> 9
aatacgactc actataggg                                19

<210> 10
<211> 24
<212> DNA
<213> Artificial Sequence

<220>
<223> Primer A1 for sequencing

<400> 10
tgccagggag tcaagtgatt tttg                          24

<210> 11
<211> 20
<212> DNA
<213> Artificial Sequence

<220>
<223> Primer A2 for sequencing

<400> 11
tttgtggcca ccctgctatt                                20

<210> 12
<211> 21
<212> DNA
<213> Artificial Sequence

<220>
<223> Primer F1 for sequencing

<400> 12
ctggaacctg cacaagagga t                              21

<210> 13
<211> 20
<212> DNA
<213> Artificial Sequence

<220>
<223> Primer F2 for sequencing

<400> 13
ctgggccaga tgaacgagtt                              20

<210> 14
<211> 20
<212> DNA
<213> Artificial Sequence

<220>
<223> Primer F3 for sequencing

<400> 14
ttgccttcat gttgcctgtg                              20

<210> 15
<211> 24
<212> DNA
<213> Artificial Sequence

<220>
<223> Primer B1 for sequencing

<400> 15
agggcacttt ctcctgggtt acct                                24

<210> 16
<211> 20
<212> DNA
<213> Artificial Sequence

<220>
<223> Primer B2 for sequencing

<400> 16
gggtcatttt ggggtgtgaa                                20

<210> 17
<211> 25
<212> DNA
<213> Artificial Sequence

<220>
<223> Primer B8 for sequencing

<400> 17
gcagtgggag gttgctcaag accag                                25

<210> 18
<211> 21
<212> DNA
<213> Artificial Sequence

<220>
<223> Primer R1 for sequencing

<400> 18
atcctcttgt gcaggttcca g                                21

<210> 19
<211> 20
<212> DNA
<213> Artificial Sequence

<220>
<223> Primer R2 for sequencing

<400> 19
aactcgttca tctggcccag                                20

<210> 20
<211> 20
<212> DNA
<213> Artificial Sequence

<220>
<223> Primer R3 for sequencing

<400> 20
aatagcaggg tggccacaaa                                20

<210> 21
<211> 20
<212> DNA
<213> Artificial Sequence

<220>

<223> Primer R4 for sequencing

<400> 21
cacaggcaac atgaaggcaa                    20

<210> 22
<211> 2320
<212> DNA
<213> Homo sapiens

<400> 22
gcctctagct gcccctcact cgtctcgccc gccagtctcc ctcccgcgcg atggcctcgg    60
cgctgagcta tgtctccaag ttcaagtcct tcgtgatctt gttcgtcacc ccgctcctgc   120
tgctgccact cgtcattctg atgcccgcca agtttgtcag gtgtgcctac gtcatcatcc   180
tcatggccat ttactggtgc acagaagtca tccctctggc tgtcacctct ctcatgcctg   240
tcttgctttt cccactcttc cagattctgg actccaggca ggtgtgtgtc cagtacatga   300
aggacaccaa catgctgttc ctgggcggcc tcatcgtggc cgtggctgtg gagcgctgga   360
acctgcacaa gaggatcgcc ctgcgcacgc tcctctgggt gggggccaag cctgcacggc   420
tgatgctggg cttcatgggc gtcacagccc tcctgtccat gtggatcagt aacacggcaa   480
ccacggccat gatggtgccc atcgtggagg ccatattgca gcagatggaa gccacaagcg   540
cagccaccga ggccggcctg gagctggtgg acaagggcaa ggccaaggag ctgccaggga   600
gtcaagtgat ttttgaaggc cccactctgg ggcagcagga agaccaagag cggaagaggt   660
tgtgtaaggc catgaccctg tgcatctgct acgcggccag catcgggggc accgccaccc   720
tgaccgggac gggacccaac gtggtgctcc tgggccagat gaacgagttg tttcctgaca   780
gcaaggacct cgtgaacttt gcttcctggt ttgcatttgc ctttcccaac atgctggtga   840
tgctgctgtt cgcctggctg tggctccagt ttgtttacat gagattcaat tttaaaaagt   900
cctggggctg cgggctagag agcaagaaaa acgagaaggc tgccctcaag gtgctgcagg   960
aggagtaccg gaagctgggg cccttgtcct tcgcggagat caacgtgctg atctgcttct  1020
tcctgctggt catcctgtgg ttctcccgag accccggctt catgcccggc tggctgactg  1080
ttgcctgggt ggagggtgag acaaagtatg tctccgatgc cactgtggcc atctttgtgg  1140
ccaccctgct attcattgtg ccttcacaga agcccaagtt taacttccgc agccagactg  1200
aggaagaaag gaaaactcca ttttatcccc ctcccctgct ggattggaag gtaacccagg  1260
agaaagtgcc ctggggcatc gtgctgctac tagggggcgg atttgctctg gctaaaggat  1320
ccgaggcctc ggggctgtcc gtgtggatgg ggaagcagat ggagcccttg cacgcagtgc  1380
ccccggcagc catcaccttg atcttgtcct tgctcgttgc cgtgttcact gagtgcacaa  1440
gcaacgtggc caccaccacc ttgttcctgc ccatctttgc ctccatgtct cgctccatcg  1500
gcctcaatcc gctgtacatc atgctgccct gtaccctgag tgcctccttt gccttcatgt  1560
tgcctgtggc caccccctcca aatgccatcg tgttcaccta tgggcacctc aaggttgctg  1620
acatggtgaa aacaggagtc ataatgaaca taattggagt cttctgtgtg tttttggctg  1680
tcaacacctg ggacgggcc atatttgact tggatcattt ccctgactgg gctaatgtga  1740
cacatattga gacttaggaa gagccacaag accacacaca cagcccttac cctcctcagg  1800
actaccgaac cttctggcac accttgtaca gagttttggg gttcacaccc caaaatgacc  1860
caacgatgtc cacacaccac caaaacccag ccaatgggcc acctcttcct ccaagcccag  1920
atgcagagat ggtcatgggc agctggaggg taggctcaga aatgaaggga acccctcagt  1980
gggctgctgg acccatcttt cccaagcctt gccattatct ctgtgaggga ggccaggtag  2040
ccgagggatc aggatgcagg ctgctgtacc cgctctgcct caagcatccc ccacacaggg  2100
ctctggtttt cactcgcttc gtcctagata gtttaaatgg gaatcggatc ccctggttga  2160
gagctaagac aaccacctac cagtgcccat gtcccttcca gctcaccttg agcagcctca  2220
gatcatctct gtcactctgg aagggacacc ccagccaggg acggaatgcc tggtcttgag  2280
caacctccca ctgctggagt gcgagtggga atcagagcct                        2320

<210> 23
<211> 572
<212> PRT
<213> Mus spp

<400> 23
Met Asp Ser Ala Lys Thr Cys Val Thr Lys Phe Lys Ser Phe Ala Ile
                5                   10                  15
Leu Leu Phe Thr Pro Ile Leu Met Leu Pro Leu Val Ile Leu Ile Pro
            20                  25                  30
Asp Lys Phe Ala Arg Cys Ala Tyr Val Ile Val Ile Met Ala Val Tyr
        35                  40                  45
Trp Cys Thr Asp Val Ile Pro Val Ala Val Thr Ser Leu Leu Pro Val
    50                  55                  60
Leu Leu Phe Pro Leu Leu Lys Val Leu Asp Ser Lys Gln Val Cys Ile
65                  70                  75                  80
Gln Tyr Met Lys Asp Thr Asn Met Leu Phe Leu Gly Ser Leu Ile Val

85                  90                  95
Ala Val Ala Val Glu Arg Trp Lys Leu His Lys Arg Val Ala Leu Arg
            100                 105                 110
Met Leu Leu Phe Val Gly Thr Lys Pro Ser Arg Leu Met Leu Gly Phe
        115                 120                 125
Met Phe Val Thr Ala Phe Leu Ser Met Trp Ile Ser Asn Thr Ala Ala
    130                 135                 140
Thr Ala Met Met Ile Pro Ile Val Glu Ala Met Leu Gln Gln Met Ile
145                 150                 155                 160
Ala Ala Asn Thr Ala Val Glu Ala Ser Leu Gly Thr Leu Glu Leu Leu
                165                 170                 175
Asp Lys Asn Lys Thr Ser Glu Leu Pro Gly Ser Gln Val Val Phe Glu
            180                 185                 190
Asp Pro Asn Val Gln Glu Gln Glu Asp Glu Glu Thr Lys Asn Met Tyr
        195                 200                 205
Lys Ala Met His Leu Cys Val Cys Tyr Ser Ala Ser Ile Gly Gly Thr
    210                 215                 220
Ala Thr Leu Thr Gly Thr Gly Pro Asn Val Val Leu Leu Gly Gln Met
225                 230                 235                 240
Gln Glu Leu Phe Pro Asp Ser Lys Asp Val Leu Asn Tyr Ala Ser Trp
                245                 250                 255
Phe Gly Phe Ala Phe Pro Asn Met Val Met Met Leu Val Leu Ala Trp
            260                 265                 270
Leu Trp Leu Gln Cys Leu Tyr Met Arg His Asn Leu Lys Lys Thr Cys
        275                 280                 285
Ile Cys Cys Gly Glu Lys Lys Arg Asp Thr Glu Lys Ile Ala Tyr Lys
    290                 295                 300
Val Leu Asn Glu Glu Tyr Gln Lys Leu Gly Ser Leu Ser Tyr Pro Glu
305                 310                 315                 320
Cys Asn Val Leu Phe Cys Phe Thr Leu Leu Val Ile Leu Trp Phe Ser
                325                 330                 335
Arg Asp Pro Gly Phe Met Pro Gly Trp Leu Ser Phe Ala Trp Val Glu
            340                 345                 350
Gly Asn Thr Val His Ile Thr Asp Ala Thr Val Ala Ile Phe Val Ala
        355                 360                 365
Ile Leu Leu Phe Ile Ile Pro Ser Gln Lys Pro Lys Phe Asn Phe Ser
    370                 375                 380
Ser Gln Thr Glu Glu Glu Arg Lys Thr Pro Phe Tyr Pro Pro Ala Leu
385                 390                 395                 400
Leu Asp Trp Lys Val Ala Gln Glu Lys Val Pro Trp Asp Ile Val Leu
                405                 410                 415
Leu Leu Gly Gly Gly Phe Ala Met Ala Lys Gly Cys Glu Thr Ser Gly
            420                 425                 430
Leu Ser Lys Trp Met Ala Ala Gln Met Glu Pro Leu Arg Leu Val Lys
        435                 440                 445
Pro Ala Val Ile Thr Leu Ile Leu Ser Cys Leu Val Ala Met Thr Thr
    450                 455                 460
Glu Cys Thr Ser Asn Val Ala Thr Thr Thr Leu Phe Leu Pro Ile Phe
465                 470                 475                 480
Ala Ser Met Ala Arg Ser Ile Gly Ile His Pro Leu Tyr Val Met Ile
                485                 490                 495
Pro Cys Thr Met Ser Ala Ser Leu Ala Phe Met Leu Pro Val Ala Thr
            500                 505                 510
Pro Pro Asn Ala Ile Val Phe Ala Tyr Gly His Leu Arg Val Val Asp
        515                 520                 525
Met Met Lys Thr Gly Leu Ile Met Asn Phe Val Gly Ile Leu Ser Val
    530                 535                 540
Phe Leu Ser Val Asn Thr Trp Gly Arg Ala Met Phe Asn Leu Asp Asn
545                 550                 555                 560
Phe Pro Asp Trp Ala Asn Ser Thr Ser Val Asn Thr
                565                 570

<210> 24
<211> 1716
<212> DNA
<213> Mus spp

<400> 24

atggattcgg cgaagacttg tgtgaccaag ttcaagtcct ttgcgatttt gctcttcacc 60
ccgatcctga tgcttccact cgtcattctg atacctgaca agtttgccag gtgtgcctat 120
gttatagtca ttatggctgt ctactggtgc acagatgtca tcccagtggc tgttacctcc 180
ctcctgcctg tcttactctt cccacttttg aaggttctgg actccaagca ggtatgtatc 240
caatacatga aggacaccaa catgctgttc ctgggcagtc tcattgtggc tgtggctgtg 300
gaacgctgga aacttcataa gagggttgcc ctgagaatgc tgctctttgt ggggaccaag 360
ccctcacggc tgatgctggg ctttatgttt gtcacggcct tcctgtccat gtggatcagc 420
aatactgccg ccacagccat gatgataccc attgtggagg ccatgctgca gcaaatgata 480
gccgccaata cagctgtgga ggccagcctg ggacactgga agctgctgga caagaacaag 540
accagcgagt tgccaggaag ccaggtggta tttgaagacc ccaatgtgca ggagcaggaa 600
gacgaagaaa caaagaatat gtacaaggct atgcacctat gtgtttgcta ttcagccagc 660
attgggggta cagccacctt gaccgggacg ggacccaacg tggtgctcct gggccagatg 720
caggaattgt ttcctgacag taaagatgtc ctgaactatg catcttggtt tggatttgcc 780
ttccccaaca tggtgatgat gctggtgctg gcctggctgt ggctccagtg cttgtacatg 840
agacacaatt taaaaaaaac ttgcatctgc tgtggggaga agaagaggga caccgagaag 900
attgcctaca aagtgctgaa cgaggagtac cagaagctgg ggtccttgag ctaccctgaa 960
tgcaacgtgc tcttttgctt caccctactt gtcatcctgt ggttctcccg agaccccggc 1020
ttcatgcctg gctggctgtc attcgcctgg gtcgagggaa acaccgttca tatcacagat 1080
gccacagtgg ccatctttgt ggccattttg cttttcatca taccttcaca aaagcccaag 1140
ttcaacttca gcagccagac tgaggaagaa aggaaaactc cgttctaccc cccagcactg 1200
ctggattgga aagtcgccca agagaaagtg ccctgggaca tcgtgctgct cctgggggga 1260
gggtttgcta tggcaaaagg atgtgagaca tcagggctct cgaagtggat ggcagcacag 1320
atggaaccct tgagattagt gaaacctgct gtcattacct tgatcttgtc ctgtcttgtt 1380
gcaatgacca cagagtgcac aagtaacgtg gccactacca ccctgttcct gcctatcttt 1440
gcctccatgg ctcgttccat tggtatccat cctctgtatg tcatgattcc ctgtaccatg 1500
agtgcttcac ttgccttcat gttgcctgtg gccaccccac cgaatgccat cgtgtttgcc 1560
tacggacacc tcagagttgt tgacatgatg aaaacaggat tgataatgaa cttcgttgga 1620
atcctatctg tgtttctgtc agtcaacacc tggggtcggg ctatgtttaa cttggataac 1680
ttccccgact gggcaaattc aacaagtgtt aacact 1716

<210> 25
<211> 27
<212> DNA
<213> Artificial Sequence

<220>
<223> Primer

<400> 25
ccatcctaat acgactcact atagggc 27

<210> 26
<211> 24
<212> DNA
<213> Artificial Sequence

<220>
<223> Primer

<400> 26
aagagcagca ttctcagggc aacc 24

<210> 27
<211> 23
<212> DNA
<213> Artificial Sequence

<220>
<223> Primer

<400> 27
actcactata gggctcgagc ggc 23

<210> 28
<211> 24
<212> DNA
<213> Artificial Sequence

<220>
<223> Primer

<400> 28
gacaggcagg agggaggtaa cagc                                              24

<210> 29
<211> 24
<212> DNA
<213> Artificial Sequence

<220>
<223> Primer

<400> 29
gttttctccc tccagtcagt ctcc                                              24

<210> 30
<211> 24
<212> DNA
<213> Artificial Sequence

<220>
<223> Primer

<400> 30
ggtgtgccag aaggttcata gtcc                                              24

<210> 31
<211> 24
<212> DNA
<213> Artificial Sequence

<220>
<223> Primer

<400> 31
aggaagccag gtggtatttg aaga                                              24

<210> 32
<211> 23
<212> DNA
<213> Artificial Sequence

<220>
<223> Primer

<400> 32
gcagcacaga tggaaccctt gag                                               23

<210> 33
<211> 24
<212> DNA
<213> Artificial Sequence

<220>
<223> Primer

<400> 33
cctgcacatt ggggtcttca aata                                              24

<210> 34
<211> 23
<212> DNA
<213> Artificial Sequence

<220>
<223> Primer

<400> 34
ctcaagggtt ccatctgtgc tgc                                              23

<210> 35
<211> 356
<212> DNA
<213> Mus spp

<400> 35
gttttctccc tccagtcagt ctccctttca cgcgatggat tcggcgaaga cttgtgtgac      60
caagttcaag tcctttgcga ttttgctctt caccccgatc ctgatgcttc cactcgtcat     120
tctgatacct gacaagtttg ccaggtgtgc ctatgttata gtcattatgg ctgtctactg     180
gtgcacagat gtcatcccag tggctgttac ctccctcctg cctgtcttac tcttcccact     240
tttgaaggtt ctggactcca agcaggtatg tatccaatac atgaaggaca ccaacatgct     300
gttcctgggc agtctcattg tggctgtggc tgtggaacgc tggaaacttc ataaga         356

<210> 36
<211> 1826
<212> DNA
<213> Mus spp

<400> 36
   gttttctccc tccagtcagt ctccctttca cgcgatggat tcggcgaaga cttgtgtgac      60
   caagttcaag tcctttgcga ttttgctctt caccccgatc ctgatgcttc cactcgtcat     120
   tctgatacct gacaagtttg ccaggtgtgc ctatgttata gtcattatgg ctgtctactg     180
   gtgcacagat gtcatcccag tggctgttac ctccctcctg cctgtcttac tcttcccact     240
   tttgaaggtt ctggactcca agcaggtatg tatccaatac atgaaggaca ccaacatgct     300
   gttcctgggc agtctcattg tggctgtggc tgtggaacgc tggaaacttc ataagagggt     360
   tgccctgaga atgctgctct ttgtggggac caagccctca cggctgatgc tgggctttat     420
   gtttgtcacg gccttcctgt ccatgtggat cagcaatact gccgccacag ccatgatgat     480
   acccattgtg gaggccatgc tgcagcaaat gatagccgcc aatacagctg tggaggccag     540
   cctggggaca ctggagctgc tggacaagaa caagaccagc gagttgccag gaagccaggt     600
   ggtatttgaa gaccccaatg tgcaggagca ggaagacgaa gaaacaaaga atatgtacaa     660
   ggctatgcac ctatgtgttt gctattcagc cagcattggg ggtacagcca ccttgaccgg     720
   gacgggaccc aacgtggtgc tcctgggcca gatgcaggaa ttgtttcctg acagtaaaga     780
   tgtcctgaac tatgcatctt ggtttggatt tgccttcccc aacatggtga tgatgctggt     840
   gctggcctgg ctgtggctcc agtgcttgta catgagacac aatttaaaaa aaacttgcat     900
   ctgctgtggg gagaagaaga gggacaccga gaagattgcc tacaaagtgc tgaacgagga     960
   gtaccagaag ctggggtcct tgagctaccc tgaatgcaac gtgctctttt gcttcaccct    1020
   acttgtcatc ctgtggttct cccgagaccc cggcttcatg cctggctggc tgtcattcgc    1080
   ctgggtcgag ggaaacaccg ttcatatcac agatgccaca gtggccatct ttgtggccat    1140
   tttgcttttc atcatacctt cacaaaagcc caagttcaac ttcagcagcc agactgagga    1200
   agaaaggaaa actccgttct accccccagc actgctggat tggaaagtcg cccaagagaa    1260
   agtgccctgg gacatcgtgc tgctcctggg gggagggttt gctatggcaa aaggatgtga    1320
   gacatcaggg ctctcgaagt ggatggcagc acagatggaa cccttgagat tagtgaaacc    1380
   tgctgtcatt accttgatct tgtcctgtct tgttgcaatg accacagagt gcacaagtaa    1440
   cgtggccact accaccctgt tcctgcctat ctttgcctcc atggctcgtt ccattggtat    1500
   ccatcctctg tatgtcatga ttccctgtac catgagtgct tcacttgcct tcatgttgcc    1560
   tgtggccacc ccaccgaatg ccatcgtgtt tgcctacgga cacctcagag ttgttgacat    1620
   gatgaaaaca ggattgataa tgaacttcgt tggaatccta tctgtgtttc tgtcagtcaa    1680
   cacctggggt cgggctatgt ttaacttgga taacttcccc gactgggcaa attcaacaag    1740
   tgttaacact taggaagagc cgcaagagca caagcatgcc ccccccaccc caaccctttt    1800
   gaggactatg aaccttctgg cacacc                                         1826

<210> 37
<211> 21
<212> DNA
<213> Artificial Sequence

<220>
<223> Primer

<400> 37
actggagctg ctggacaaga a                                                21

<210> 38
<211> 19

<212> DNA
<213> Artificial Sequence

<220>
<223> Primer

<400> 38
tcctgctcct gcacattgg 19

<210> 39
<211> 25
<212> DNA
<213> Artificial Sequence

<220>
<223> Probe

<400> 39
aagaccagcg agttgccagg aagcc 25

<210> 40
<211> 37
<212> DNA
<213> Artificial Sequence

<220>
<223> Primer

<400> 40
ccataagctt gtcgacatgg cctcggcgct gagctat 37

<210> 41
<211> 37
<212> DNA
<213> Artificial Sequence

<220>
<223> Primer

<400> 41
agcttctaga actagtctaa gtctcaatat gtgtcac 37

<210> 42
<211> 18
<212> DNA
<213> Artificial Sequence

<220>
<223> Primer

<400> 42
tagaaggcac agtcgagg 18